Europäisches Patentamt

European Patent Office

Office européen des brevets

(19)

(11) Publication number: **0 367 063**
**A1**

## EUROPEAN PATENT APPLICATION

(12)

(21) Application number: **89119642.0**

(22) Date of filing: **23.10.89**

(51) Int. Cl.5 **C12N 15/21 , C12P 21/00 , A61K 37/02 , C07K 13/00**

(30) Priority: **26.10.88 US 262870**

(43) Date of publication of application:
**09.05.90 Bulletin 90/19**

(84) Designated Contracting States:
**AT BE CH DE ES FR GB GR IT LI LU NL SE**

(71) Applicant: **THE CURATORS OF THE UNIVERSITY OF MISSOURI**
**215 University Hall**
**Columbia Missouri 65211(US)**

(72) Inventor: **Roberts, Michael R., Dr.**
**2213 Hominy Branch Court**
**Columbia, MO 65201(US)**
Inventor: **Imakawa, Kazuhiko, Dr.**
**101 South Rock Road, No. 10**
**Derby Kansas 67037(US)**

(74) Representative: **Zumstein, Fritz, Dr. et al**
**Dr. F. Zumstein Dipl.-Ing. F. Klingseisen**
**Bräuhausstrasse 4**
**D-8000 München 2(DE)**

(54) **Isolation and cloning of complementary DNA for gene coding of bovine trophoblast protein-1.**

(57) The present invention relates to DNA sequences encoding bTP-1, to isolation and identification thereof and to the construction of recombinant DNA expression vehicles, particularly complementary DNA (cDNA) containing such DNA sequences operably linked to expression-effecting promoters sequences and to the expression vehicles so constructed. In another aspect, the present invention relates to cDNA for bTP-1 and the isolation thereof. In yet other aspects, the invention relates to essentially pure bovine trophoblast protein-1, to means and methods of providing this protein by recombinant DNA technology and of converting recombinant bTP-1 to novel entities, such as pharmaceutical compositions, useful for the prophylactic of therapeutic treatment of other species of warm-blooded animals, including humans.

FIG.7

EP 0 367 063 A1

# ISOLATION AND CLONING OF COMPLEMENTARY DNA FOR GENE CODING OF BOVINE TROPHOBLAST PROTEIN-1

## FIELD OF THE INVENTION

The present invention relates to the field of recombinant DNA technology and to means and methods utilizing such technology in the discovery of the DNA sequence and deduced amino acids sequence for the bovine embryo protein bovine trophoblast protein-1 (bTP-1), which is an interferon within the alpha family of leukocyte interferons ($\alpha$-interferon or IFN-$\alpha$).

More particularly, the present invention relates to DNA sequences encoding bTP-1, to isolation and identification thereof and to the construction of recombinant DNA expression vehicles, particularly complementary DNA (cDNA) containing such DNA sequences operably linked to expression-effecting promoters sequences and to the expression vehicles so constructed. In another aspect, the present invention relates to cDNA for bTP-1 and the isolation thereof. In yet other aspects, the invention relates to essentially pure bovine trophoblast protein-1, to means and methods of providing this protein by recombinant DNA technology and of converting recombinant bTP-1 to novel entities, such as pharmaceutical compositions, useful for the prophylactic of therapeutic treatment of other species of warm-blooded animals, including humans. In preferred embodiments, this invention provides particular expression vehicles that ensure that bTP-1 is produced and secreted from the host cell. In addition, this invention relates to various processes useful for producing said DNA sequences, expression vehicles, host culture systems and end products and entities thereof and to specific and associated embodiments thereof.

The present invention arises from the discovery of the DNA sequence and deduced amino acid sequence encoding bTP-1. In addition, the present invention provides the information that bTP-1 belongs to the family of interferon-$\alpha_{II}$. The availability of the protein in large quantities will enable the use thereof in the following applications:

(1) Enhancement of the success of embryo transfers in cattle and other farm species when the bTp-1 protein is introduced into the uterus of the recipient animal or injected parenterally, preferably intramuscularly or subcutaneously coincident with and subsequent to the time of transfer.

(2) To promote fertility in cattle by injecting them with the bTp-1 protein at the time of maternal recognition of pregnancy (e.g. days 15-25 in cattle) for usually at this time, high rates of embryonic loss occur. These losses are believed to result because the embryo does not produce the protein early enough or in sufficient quantities. This failure normally leads to loss of function of the corpus luteum, a termination of progesterone production and a loss of the pregnancy.

(3) The treatment of other causes of infertility in cattle such as "short cycles".

(4) The gene for this protein can be introduced into fertilized eggs to produce transgenic animals with extra copies of the gene, thereby providing enhanced fertility.

(5) The protein is useful in the treatment of viral diseases in farm species.

The publications used to illustrate the technological background of the present invention are for convenience numerically referenced by the following text and respectively groups in the appendant bibliography.

## BACKGROUND OF THE INVENTION

Bovine trophoblast protein-1 (bTP-1) is a major product of the bovine conceptus which is secreted at a time coinciding with the period of maternal recognition of pregnancy in the cow (1,2). It has been implicated as a paracrine hormone targeted to the maternal uterus which interferes with the synthesis and release of prostaglandin $F_{2\alpha}$, the presumed luteolysin (3,4). Previous methods for producing bTP-1 involved collecting embryos from cows, culturing these embryos and purifying the proteins from the culture medium by a variety of tedious procedures (2) which until now have not been successful in producing bTP-1 in more than $\mu$g amounts. The procedures are also very expensive, requiring large numbers of animals. Labor costs are high because the animals have to be bred under controlled conditions, the embryos obtained after slaughtering, cultures set up and the protein purified. The amount of bTP-1 obtained until now has been insufficient to carry out experiments on the characterization of biological properties of the purified component. The application of recombinant DNA technology as an effective way in providing larger quantities of bTP-1 was a long felt need.

In accordance with the present invention, it has been found that bTP-1 is shown to have about 50 % identity in the amino acid sequence to $\alpha$-interferons of a variety of mammalian species and about 70 % identity to a bovine IFN-$\alpha_{II}$. According to the present invention bTP-1 provides antiviral activity and inhibits the incorporation of $^3$H-thymidine into ovine lymphocytes stimulated with mitogens. Its antiviral activity approaches that of human leukocyte IFN and recombinant IFN-$\alpha_I$.

## SUMMARY OF THE INVENTION

The present invention comprises the cDNA for the bTP-1 protein.

The present invention is further directed to replicable DNA expression vehicles harboring gene sequences encoding bTP-1 in expressible form.

The present invention further comprises essentially pure bTP-1 protein and the means and methods of its production, including bovine trophoblast protein-1 prepared by means of recombinant DNA technology, having substantially the properties of the highly purified naturally occurring protein.

The present invention is further directed to replicable DNA expression vehicles harboring gene sequences encoding bTP-1 in expressible form, preferably to recombinant vectors adapted for transformation of a suitable host cell, especially plasmids adapted for the transformation of bacterial, mammalian and/or yeast cells. The process of preparing bovine trophoblast protein-1 which comprises the steps of: (a) introducing a recombinant vector comprising a DNA sequence capable of expressing bovine trophoblast protein-1 into a suitable host cell, (b) culturing the resultant transformed host cell in a suitable culture medium, and (c) isolating the recombinant bovine trophoblast protein-1 from the cultured cells.

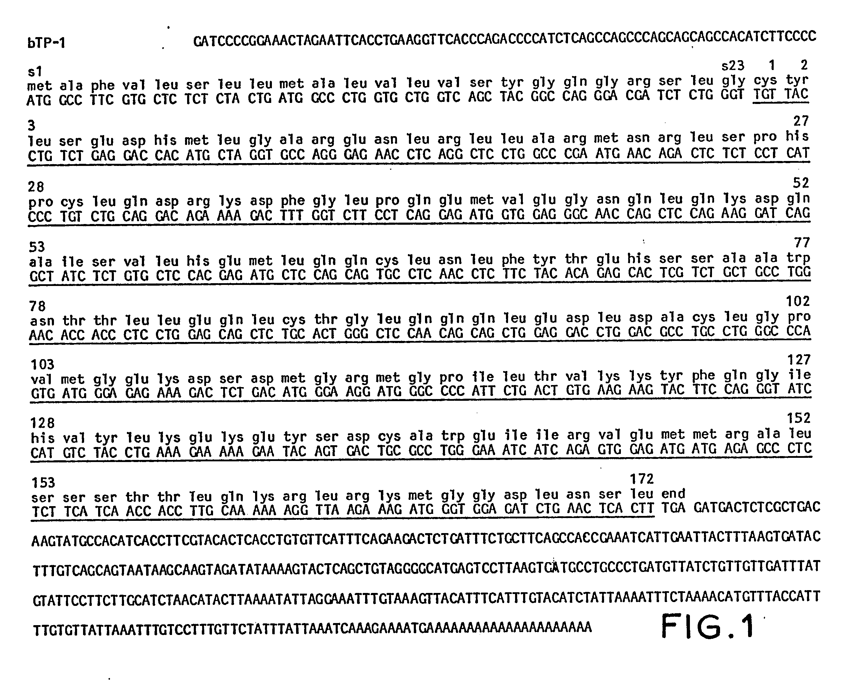

The present invention is further directed to a recombinant DNA sequence capable of expressing a bovine trophoblast protein-1 in a suitable host cell and a DNA sequence encoding a bovine trophoblast protein-1. The present invention still further relates to a DNA sequence and polypeptide shown in Figure 1 or to a functional variation thereof.

Further, the present invention is directed to microorganism and cell cultures transformed with the expression vehicles described and microbial strains or mammalian or yeast cell cultures capable of producing bTP-1.

The production of bTP-1 is based on methods of the recombinant DNA techniques. Particular embodiments include the following:

(1) The cDNA is modified by insertion of an ATG "start" codon upstream of the TGT codon for position 1 cysteine of the mature protein.

The mammalian signal sequence of bTP-1 is not expressed in the bacterial system. The bTP-1 cDNA can be modified by including a signal sequence for E. coli and/or by adjusting the upstream 5'-sequence to the preferred codon usage of E. coli.

(2) Such modified cDNA constructs are introduced into a recombinant vector adapted for transformation of a suitable host cell. Suitable vectors comprise a regulatory sequence, preferably strong promoters, capable of directing expression of foreign genes in the host operably linked to the structural gene. Recombinant vectors are chosen which are plasmids adapted for transformation of a bacterial host, a mammalian host or a yeast cell. For example, pBR vectors with trp or lac promoters can be constructed with suitable restriction sites to allow insertion of the particular cDNA construct.

(3) The pBR vector with the inserted cDNA is introduced into a microbial cell, for example into E. coli DH1 or one of its derivatives by standard transformation procedures, e.g. by the calcium chloride method described by Maniatis et al. (21).

(4) Successful transformants are isolated and established DNA sequencing used to confirm that the construct is in the correct orientation and alignment.

(5) Recombinant pBR plasmids with the correctly oriented insert are isolated from transformed cells and introduced into other microbial cells particularly useful for protein production, for example into corresponding E. coli strains, which express the protein in large amounts after induction, e.g. D112, JM103.

(6) A suitable cell line is used to synthesize bTP-1. In the case of vectors with the trp construct, cells will be grown in the absence of L-tryptophan. In the case of the lac promotor, cells are induced with isopropylthiogalactoside (IPTG).

(7) Cells are lysed to recover the recombinant bTP-1 which is solubilized and carefully renatured before purification. Preferably the procedure will exploit the isolation of inclusion bodies from the cells. Recombinant proteins are frequently diverted as almost pure products into these structures and can be renatured after solubilization and refolding. These renaturation techniques are well known to the skilled artisan.

As an alternative to the above procedure using microbial cells suitable constructs can be prepared for transfer to a cultured mammalian cell, such as the CHO cell line K1. In this case the DNA coding the mammalian signal sequence is retained. The constructs preferably contain an appropriate selectable marker so that transformants are recognized. Lines expressing bTP-1 are isolated and used for large-scale preparation of the protein which is isolated from the medium.

The present invention is additionally directed to a method for enhancing fertility in mammals comprising administerin to said mammals an effective mammalian fertility enhancing amount of a bovine trophoblast protein-1, for example the daily dosage of 0.5 to 50 mg animal, and a composition for enhancing fertility in mammals comprising an effective mammalian fertility enhancing amount of bovine trophoblast protein-1 or a protein having substantially the mammalian fertility enhancing activity of bovine trophoblast protein-1 together with non-toxic carriers or adjuvants.


BRIEF DESCRIPTION OF THE DRAWINGS

Fig. 1. shows the cDNA sequence encoding bTP-1.

Fig. 2. Comparison of the Aligned cDNA Base Sequences Representing Three bTP-1 cDNA's.
The upper of the three nuclctide sequences (sequence 1) is derived from two clones, pbtP159 representing bases 1-677 and λbTP3 representing bases 678-973. The midle sequence (sequence 2) is that derived from λbTP509. The lowest of the three nucleotide sequences (sequence 3) is derived from λbTP517. This clone represents a rearrangement in which the 3'-end of the cDNA from bases 702 to 979 with a sequence 24 A's is found at the 5'-end of the clone. These sequences are realigned in sequence 3. This results in the generation of a small 6-nucleotide gap (bases 756-761). Asterisks above each line mark differences in nucleotides between sequences 1 and 2. Asterisks shown below the sequences mark differences in nucleotides between sequences 2 and 3.

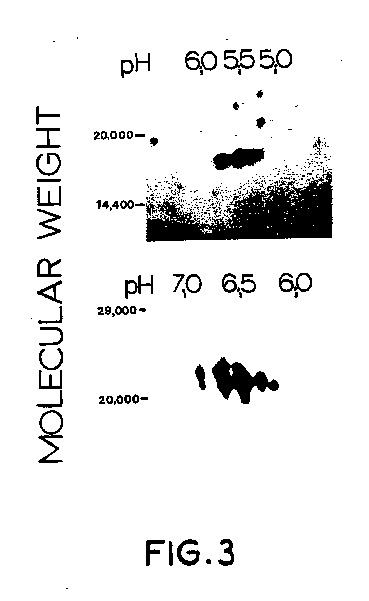

Fig.3. Two-dimensional SDS-PAGE and fluorography of [³H]-leucine-labeled ovine and bovine conceptus secretory proteins.
Day 16 ovine conceptuses and day 18 bovine conceptuses are incubated for 24 h with [³H]-leucine and the culture medium is harvested for analysis by two-dimensional SDS-PAGE(12.5 % gels) and fluorography. Ovine TP-1 (top panel) is seen as a major secretory protein with a Mr of 17,000 and consists of three isoforms (pl's 5.4 to 5.7). Bovine TP-1 (bottom panel) is seen as two Mr forms (22,000 and 24,000) and consists of at least eight isoforms (pl's 6.3 to 6.8). Only the portion of the fluorographs depicting oTP-1 and bTP-1 are presented.

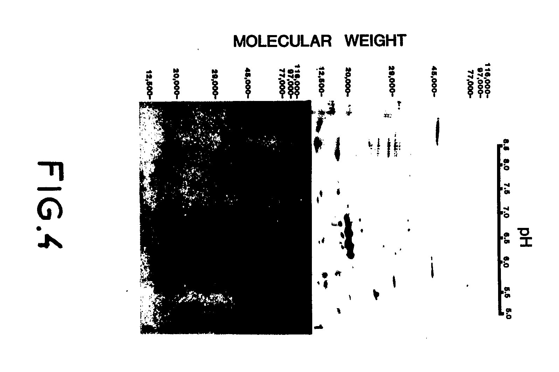

Fig.4. Two-dimensional SDS-PAGE and autoradiography of [³⁵S]-methionine-labeled proteins synthesized in the presence of bovine conceptus RNA.
In vitro translation products synthesized with wheat germ lysate in the presence of day 18 bovine conceptus polyadenylated RNA and [³⁵S]-methionine are analyzed by two-dimensional SDS-PAGE (12.5 % gels) and autoradiography. They are analyzed either as total translation products (top panel) or following immunoprecipitation with anti-oTP-1 antiserum (bottom panel).

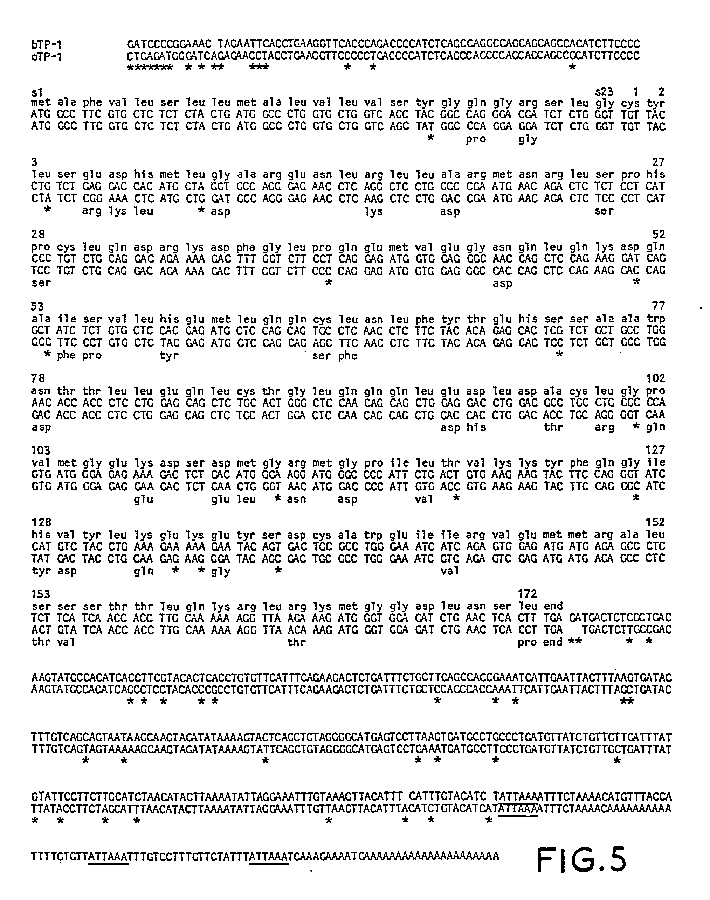

Fig. 5. Comparison of the bTP-1 and oTP-1 cDNA's and Their Dedueed Polypeptide Sequences.
The deduced amino acid sequence of bTP-1 is given above the nucleotide sequence. Amino acid residues differing in oTP-1 are indicated below the nucleotide sequence. Asterisks mark differences between the nucleotide sequences that do not result in a change of an amino acid residue.

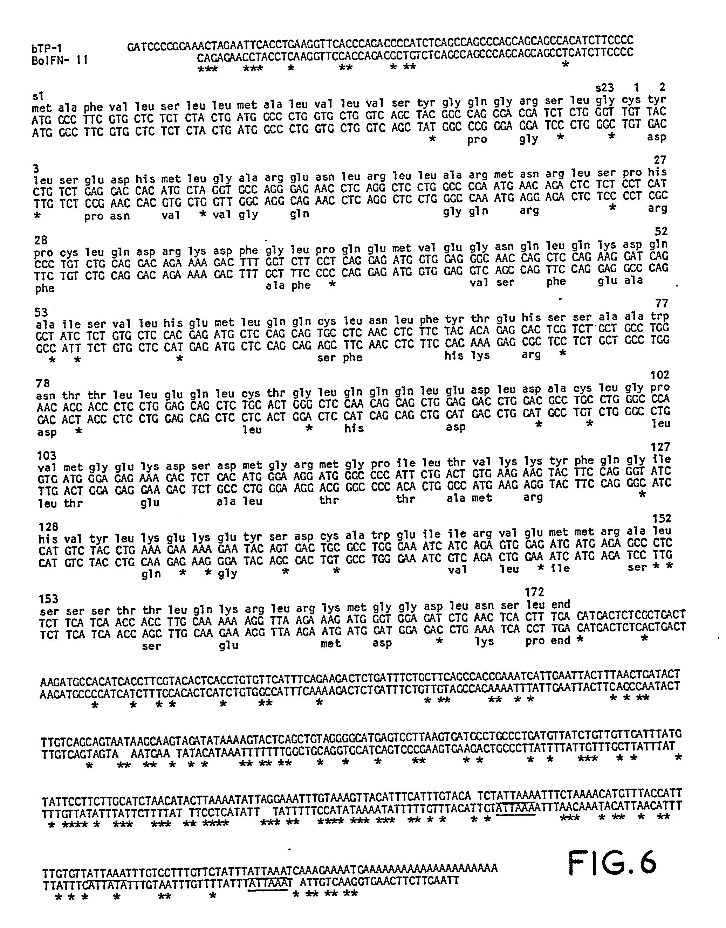

Fig. 6. Comparison of bTP-1 and Bovine IFN-α_II cDNA's and Their Deduced Polypeptide Sequences.
The deduced amino acid sequence of bTP-1 is given above the nucleotide sequence. Amino acid residues differing in bovine IFN-α_II are indicated below the nucleotide sequence. Asterisks mark differences between the nucleotide sequences that do not result in a change of an amino acid residue.

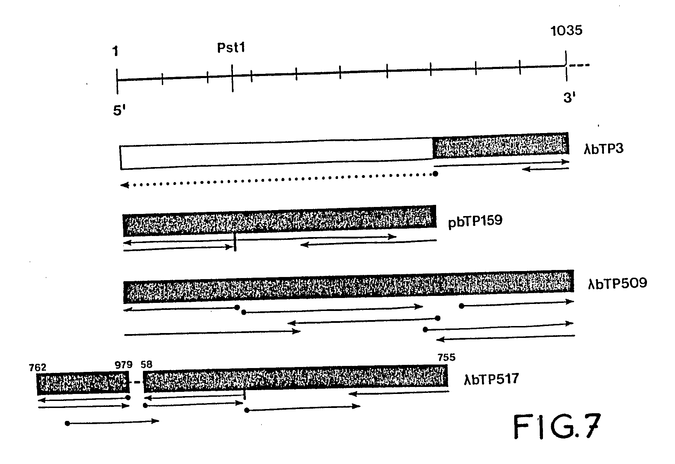

Fig.7. Sequencing Strategy for the cDNA's Whose Sequences are Represented in Fig. 3.
The upper scale represents number and relative positions of nucleotides. The clone bTP3 is isolated by immunological screening of the gtII library. The dotted line (.......) shows the extent of primer extension from an oligonucleotide complementary to a region (bases 260-280) near the 5'-end of bTP3. The clone pbTP159 represents a clone isolated from the primer extension library (in pBR322). The clones bTP509 and bTP517 are isolated from the original gtII library by rescreening with a 264 bp Pst-1 fragment from the 5' end of pbTP159. Arrows indicate the direction and extent of sequencing, the vertical line (|) is the site of cleavage by Pst-1, solid dots (.) are sites from which sequencing is initiated by uisng synthetic oliognucleotides as primers. Numbers show based positions. Note that clone λbTP517 represents a rearrangement. The 3' end of the mRNA from bases 762 to 979 with its poly(A)⁺ tail is found at the 5' end of the clone. After a stretch of 24 A's there follows a sequence representing bases 58 to 755.

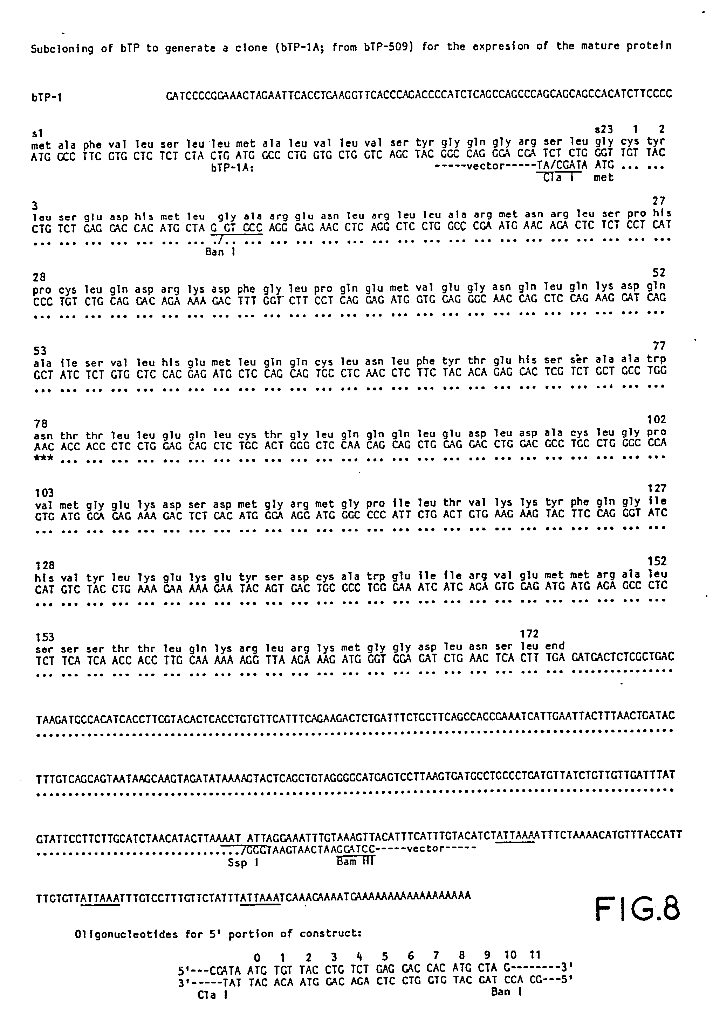

Fig. 8. Modification and sublconing of bTP-1 clone-509 to yield a construct that lacks the codons for the bTP-1 signal sequence but which contains an initiating ATG codon before the TGT (Cys1) codon. The diagram indicates the Cla I and Ban I sites into which the oligonucleotide, shown at the bottom, is inserted. The clone is further modified by creating an Ssp I blunt-ended 3'-terminal before insertion into the expression plasmid.

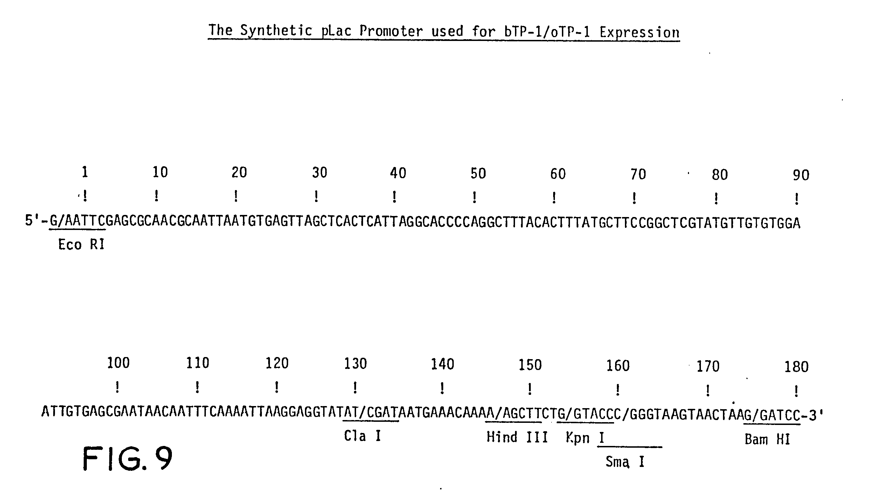

Fig. 9. The sequence of the synthetic pLac promoter. Three pairs of complementary oligonucleotides are synthesized, annealed and ligated based on complementarity of their termini. The resulting 177 base pair double-stranded fragment was then ligated into pBR322 between the Eco RI and Bam HI restriction sites to generate the expression vector designated pLac. Additional restriction sites, including the Cla I and Sma I sites used for insertion of the modified bTP-1 expression clone (Fig. 8), are shown. For details of the Lac promoter see Reference 39.

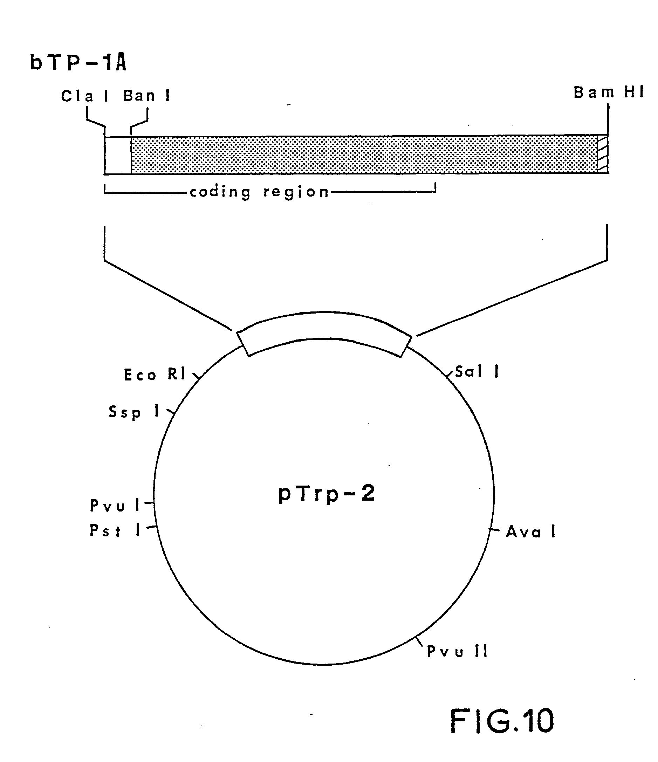

Fig. 10. Diagram showing the insertion of the bTP-1A (modified bTP-509 clone) into the pTrp-2 expression vector at its Cla I/Bam HI sites. This expression vector contains a modified Trp promoter-operator in which ribosome-binding sites are enriched with A's and T's (For details see Reference 40).

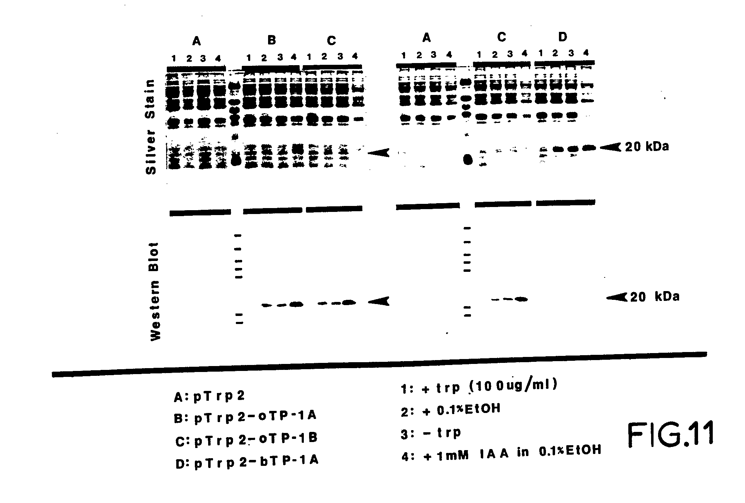

Fig. 11. Expression of bTP-1 by E. coli strain D-112 under the control of the pTrp-2 promoter. The E. coli cells at the end of the induction period are pelleted, washed and dissolved in SDS-electrophoresis buffer (32) before electrophoretic analysis in 12.5 % polyacrylamide gels. A total of about 20 μg of protein is analyzed per lane and the gels were silver stained before photography. The upper gels B and C represent induction of expression of ovine trophoblast protein-1 clones oTP-1A and oTP-1D. The lower part of the figure shows Western blots stained with an anti-oTP-1 monoclonal antibody. Gel A represents cells with plasmid vector but no insert. Gel D represents induction of bTP-1 expression. Lane 1 is uninduced control (i.e. with tryptophan in medium); Lane 2 is cells without tryptophan grown in presence of 0.1 % ethanol (the vehicle for IAA); Lane 3 shows cells grown in absence of tryptophan; Lane 4 shows cells induced by IAA (dissolved in ethanol). Note the appearance of a 20-kDa band in the induced lanes. However, this band, unlike oTP-1 (B and C) does not stain on Western blots with the anti-oTP-1 antibody. Note also the relatively poor induction of oTP-1 (which can be detected with the antibody). In D, Lane 4, the bTP-1 represented more than 28 % of cell protein.


DETAILED DESCRIPTION

Standard nomenclature and abbreviations are used fo the nucleotide bases of the DNA sequences described herein: A is for adenine; T for thymine, G for guanine; and C for cytosine. When one strand or double-stranded DNA is shown, such as cDNA sequences, it will be understood that complementarity specifies that adenine binds to thymine and guanine to cytosine: A-T (or T-A) and G-C (or C-G).


Features of the bTP-1 cDNA Base Sequences

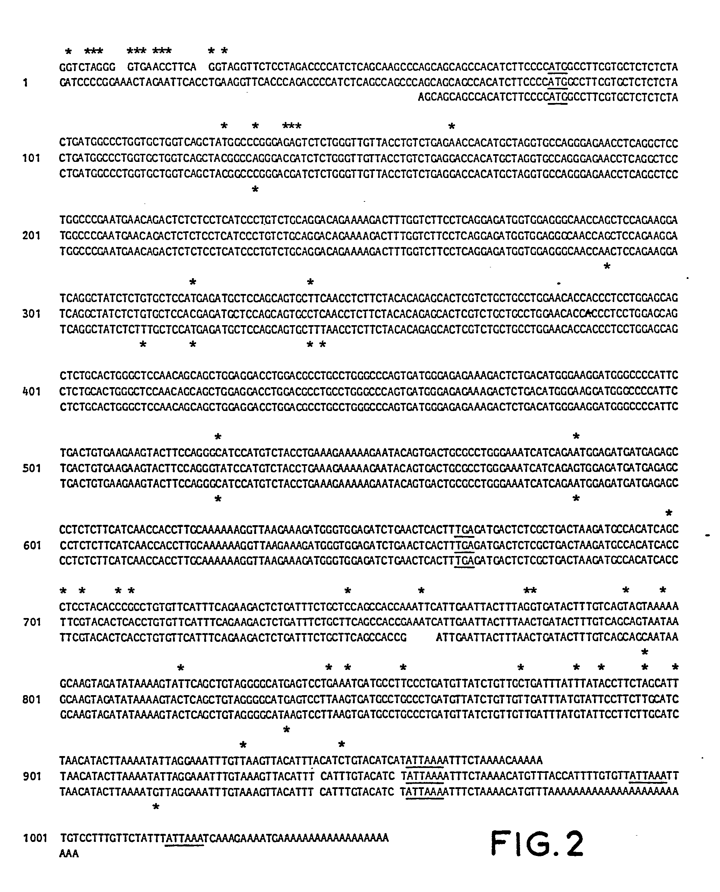

Clone bTP509 (Fig. 1) is 1035 bases in length up to the beginning of the poly(A) tail. It contains an open reading frame of 195 codons starting at the ATG codon at position 79 and ending in a termination codon TGA at position 665. The initiation codon, ATG, of this bTP-1 cDNA is immediately preceded by the sequence TCCCC which is not typical of the conensus sequence (CC$^A_G$ CC) for initiation of translation in eukaryotes (5). As with the mRNA's for the related protein ovine trophoblast protein-1 (oTP-1) (6), the hexanucleotide ATTAAA (bases 957-962, 993-998 and 1018-1023 of sequence 2) is the most likely signal for poly(A) addition to the 3'-end of the molecule. Each of the cDNA's in Fig. 2 contain such a sequence within 20 bases of the site of polyadenylation.

Comparison of aligned cDNA base sequences for different cDNA's shown in Fig. 2 indicates that each is different and arose from a distinct mRNA. The major region of dissimilarity is at the extreme 5'-ends of the molecules. The remainder of the 5'-ends, the coding regions and the 3' non-coding regions are highly conserved. If comparisons are initiated at position 58 (the start of λbTP517 - sequence 3) a total of only 31 base differences distinguish sequence 1 (λbTP3/pbTP159) and sequence 2 (λbTP509), and only 13 differences distinguish sequences 2 and 3. These base changes are scattered quite randomly through the molecules. Comparison of the sequences of three cDNA's in Fig. 2 also reveals that they differ significantly in the lengths of their 3' untranslated regions.


Features of the Deduced Amino Acid Sequences for bTP-1

The various differences in base sequences between the three cDNA clones for bTP-1 shown in Fig. 2 represent, for the most part, conservative substitutions. Together, the differences in nucleotide sequences would alter two amino acid residues within the signal peptide for the proteins coded by the three mRNA's and provide only three amino acid differences (at positions 6, 65 and 146) between sequences 1 and 2 and only three differences between 2 and 3 (at positions 56, 65 and 146). These substitutions, though minor, provide differences in overall charge among the three polypeptides and explain the presence of several isoforms.

The signal sequence for bTP-1 is 23 amino acids in length (Fig. 1). The first amino acid of the mature protein has been assigned as the cysteine residue coded by nucleotides 149-151. The residue is preceded by a glycine at position s23. The serine at position s21 then becomes the preferred small neutral amino acid as predicted by von Heijne analysis (7).

This signal sequence contains a core of hydrophobic residues (s7 to s14) and conforms to the usual length (15 to 25 residues) of leader peptides from other secreted proteins (8). The three cDNA's shown in Fig. 2 provide mature polypeptides beginning at the $Cys_1$ position that are 172 amino acids in length. Sequence 2 gives rise to a mature protein of molecular weight of 19701. Each of the bTP-1 sequences contains only a single potential glycosylation sequence, Asn-Thr-Thr, at positions 78-80.

Comparison of the Sequences of bTP-1 and oTP-1

In Fig. 5 the base sequences and the amino acid sequence for a single, full-length cDNA clone for oTP-1 (6; clone kaz6) has been aligned with the bTP-1 cDNA clone (λbTP509) from Fig. 1. The base sequences of bTP-1 and oTP-1 cDNA's are very similar (>85 % identity), with a high degree of conservation present throughout the molecules. Particularly remarkable is the high degree of sequence identity (92 %) within the 3′-regions. The only regions that are markedly dissimilar are the furthest 5′ sequences.

The deduced amino acid sequences show an overall 80 % identity. There are three substitutions within the signal sequence. Most of the other changes are scattered fairly uniformly throughout the molecules. The greatest frequencies of substitutions are found close to the $NH_2$-terminus (residues 5-10) and in three regions (residues 95-102, 107-115, 128-135) in the COOH-terminal halves of the two polypeptides. Unlike bTP-1, oTP-1 does not possess a potential glycosylation site. The latter feature has been confirmed for at least three oTP-1 cDNA's (6).

Resemblance of the Sequences of bTP-1 and oTP-1 to Those of IFN-α's

An initial computer search showed that the entire coding region of the cDNA's for bTP-1 share about 65 % base sequence identity to a series of cloned human cDNA's representing human leukocyte IFN-α's (9,10). The greatest degree of bTP-1 nucleotide sequence similarity (85 % identity) is with the bovineα$_{II}$ gene. However, there is much less conservation of nucleotide sequences (69.7 %) within the 3′ untranslated regions. This feature is illustrated in Fig. 6 in which the base sequence of the cDNA for bTP-1 is aligned with a corresponding genomic sequence of a bovine IFN-α$_{II}$ (11). Clearly the greatest difference in the two structures, other than at the immediate 5′-ends of the molecules, lies within an extremely T-rich region between bases 885 and 950. The majority of the 5′ untranslated region is well conserved, and the unusual TCCCC sequence immediately preceding the ATG initiation codon appears also to be a featue of the bovine α$_{II}$ gene. The polyadenylation signal ATTAAA is seen not only in bovine IFN-α$_{II}$ but also in several IFN-α$_I$ genes.

A comparison of the overall extent of amino acid sequence identities between bTP-1, oTP-1 and a number of selected IFN's from several mammalian species (11-15) illustrate the great diversity in amino acid sequences between IFNα's of different species. For example, pig and mouse IFN-α's differ to about the same extent as bTP-1 and hum IFN-α$_I$. However, bTP-1 and oTP-1 most clearly resemble bovine IFN-α$_{II}$ and differ from bovine IFN-α$_I$ as much as they do from the hum, rodent and pig IFN-α$_I$'s (11-15). It also appears significant that sequences for bTP-1, oTP-1 and the IFN-α$_{II}$ family of proteins are 172 amino acids in length, whereas those for the IFN-α$_I$'s are generally 166 residues long (11). Clearly bTP-1 is only distantly related to IFN-β (15).

The sequence similarities of bTP-1 to bovine IFN-α$_{II}$ occurs throughout the entire length of the polypeptides (see Fig. 6). Several amino acid residues that are found in most or all IFN-α's so far characterized are conserved in bTP-1. These include the cysteines at positions 1, 29, 99 and 139 which participate in intrachain disulfide bonds (16) and the Cys-Ala-Trp-Glu-Ile-Val-Arg sequence (residues 139-145) which are highl conserved in IFN-α's (17).

bTP-1 has Antiviral Activity

bTP-1 purified by successive ion-exchange, gel filtration and lectin affinity chromatography (on Concanavalin A-Sepharose) has a specific activity of approximately $0.3 \times 10^6$ antiviral units/mg protein when assayed in a cytopathic inhibition assay (see Table 1). This value approaches that of purified IFN-$\alpha$'s (18).

TABLE 1

| Stage of Purification | Relative Units/mg Protein |
|---|---|
| Culture Medium | $0.54 \times 10^4$ |
| DEAE-cellulose pool | $0.11 \times 10^6$ |
| Sephacryl S-200 pool | $0.38 \times 10^6$ |

Day 19 bovine embryos are cultured in MEM medium (1). After 24 h the medium is dialyzed (1) and assayed for antiviral activity in a cytopathic reduction assay that employs GBK-2 cells and vesicular stomatitis virus (35,36). The pooled medium is subjected to DEAE ion-exchange chromatography and Sephacryl S-200 ion exchange chromatography as described by Godkin et al. (26) for purification of oTP-1. However, All buffers contained 33 % by volume ethylene glycol to reduce aggregation of bTP-1 (see 1).

Description of Modified cDNA for bTP-1

A cDNA sequence slightly modified from Fig. 1 is incorporated into a plasmid derived from pBR322 (supplied by Pharmacia). In Figure 1, the underlined sequence is necessary to code for the production of bTP-1 in a living bacterial cell. When incorporated into certain bacterial plasmids, such as pBR322, and allowed to enter a bacterial cell, the DNA provides a template for the production of bTP-1 messenger RNA which, in turn, can be translated to produce the genuine protein in large quantities.

DETAILED METHODOLOGY FOR OBTAINING AND SEQUENCING cDNA'S

Materials

Restriction and modifying enzymes are purchased from Bethesda Research Laboratories (Gaithersburg, MD), Boehringer Mannheim (Indianapolis, IN), New England Biolabs (Beverly, MA) and Seikagaku America Inc. (St. Petersburg, FL). [$\alpha$-$^{32}$P]dNTP's are obtained from Dupont NEN Research Products (Boston, MA). Nitrocellulose membranes (Schleicher & Schuell, Keene, NH) are used in all DNA and RNA transfer procedures. Transformation competent strain of Escherichia coli, JM 101· and E. coli Y 1088, Y 1089 and Y1090, the bacteriophage cloning vector λgtII and packaging extracts (Lambda gtII/Gigapack cloning kit) are purchased from Stratagene (La Jolla, CA).

Plasmid cloning vectors (pUCl3, pBR322), linkers, dNTP's and ddNTP's are purchased from Pharmacia Inc. (Piscataway, NJ). Oligo-dT cellulose and oligo-dT are purchased from Boehringer Mannheim. Immunoscreening (ProtoBlot) and nick translation kits are obtained from Promega Biotec (Madison, WI) and Amersham Corporation (Arlington Heights, IL), respectively. Oligonucleotides are synthesized at the Oligonucleotide Synthesis Facility, University of Missouri by Dr. J. Forrester. Said materials as well as all other materials are commercially and thus publicly available.

Animals and Collection of Conceptuses

Cows and heifers of Holstein, Jersey and crossbred beef breeding are artificially inseminated 12 h after the observation of behavioral estrus (day 0), and conceptuses are recovered on day 18 and 19 of pregnancy (1,2).

7

Isolation, Translation and Northern Analysis of Conceptus mRNA

Isolation and in vitro translation of bovine conceptus poly(A)[+] RNA's are described in references 2, 1 9 and 20.

For Northern blot hybridization analysis, the poly(A)[+] RNA (2 μg) is separated by formaldehyde-agarose gel electrophoresis and is transferred to a nitrocellulose filter (21). Prior to hybridization with bTP-1 cDNA probe ([32]p-labeled λbTP3) the filter is incubated at 42°C for 3 h in a solution containing: 50 % (v v) formamide; 5X SSC (1X SSC is 0.15M NaCl; 0.015M sodium citrate); 0.05 % polyvinyl pyrolidone; 0.05 % ficoll; 0.05 bovine serum albumin; 50 mM sodium phosphat (pH 6.5); 0.1 % sodium dodecyl sulfate (SDS) and 0.5 mg/ml herring sperm DNA. The filter is then hybridized at 42°C for 24 h in the above buffer which contains [32]p-labeled λbTP3 (2 x 10[5] cpm/ml). The specific activity of the cDNA probe is 2 x 10[8] cpm/μg. After hybridization, the filter is washed twice at room temperature in 5X SSC and 0.1 % SDS for 15 min each. The filter is dried briefly at room temperature and is exposed to x-ray film (Kodak, XAR) in the presence of an intensifying screen at -80°C.

Construction, Screening and Sequencing of λgtll Library

Complementary cDNA's are synthesized in vitro by using the method of Gubler and Hoffman (22), as modified by Polites and Marotti (23). The poly(A)[+] RNA is annealed to an oligo(dT) primer and used to construct a double-stranded cDNA. The cDNA is then fractioned on a column (1 x 30 cm) of agarose A-50 M. Polymers of size greater than 350 bp are methylated to protect internal EcoRi restriction sites, and EcoRI linkers are added to the cDNA before cloning into the EcoRI site of bacteriophage lambda gtll (24). The ligated DNA fragments are packaged in vitro to produce infective phage particles. The phage libraries are amplified in E. coli Y 1088 then screened in E. coli Y 1090 (24,25).

About 28,000 recombinant phages are plated with E. coli Y 1090 at a density of about 9,500 plaques per 150-mm plate. After a 3.5 h growth period at 42°C, the plates are overlaid with nitrocellulose filters saturated with 10 mM isopropylthiogalactoside (IPTG) and incubated for 3.5 additional hours at 37°C. The filters are removed from the plates and blocked by incubation in 10 mM Tris HCl (pH 8.0), 150 mM NaCl, 0.05 % Tween 20 and 20 % (v/v) fetal half serum. The filters are then incubated with the primary antibody (diluted 1:200) against oTP-1 (26) which had been passed through an E. coli Y1090 lysate column (24). The plaques containing bTP-1 cDNA are identified by using the second antibody, a sheep anti-rabbit IgG conjugated to alkaline phosphatase and color substrates: 5-bromo-4-chloro-3-indolyl phosphate (BCIP) and nitroblue tetrazolium (NBT).

In order to determine sensitivity of the above immunodetection method, various concentrations of purified oTP-1 (26) are spotted directly onto a nitrocellulose filter. Anti-oTP-1 antiserum can detect 50 pg of purified oTP-1 under these conditions. Sensitivity towards bTP-1 is approximately 5- to 10-fold less. The antiservm employed is that described by Godkin et al. (27) for immunolocalization of oTP-1 in trophoblast cells and which specifically immunoprecipitates oTP-1 from a mixture of conceptus proteins.

Twenty-three bTP-1-positive plaques are identified, and six of these are subjected to plaque purification under the same conditions as used for the identification of positive plaques with anti-oTP-1 antiserum (6). The largest bTP-1 cDNA found from immunoscreening of the λgtll library is λpTP3 (350 bp), which is ligated into the EcoRI site of the digested and dephosphorylated pUCl4 plasmid and is then transformed into E. coli JM101 by the calcium chloride method (21). Nucleotide sequences are determined directly from the plasmid (28) by employing the dideoxy chain termination method (29).

Primer Extension and Screening of pBR322 Library

In order to determine the length of bTP-1 mRNA and to obtain the 5′ regions of bTP-1 cDNA, a new cDNA library is constructed by using a primer extension method and a synthetic oligonucleotide. An oligonucleotide 5′d(GGG TGT AGG AGG CTG AGT TGG)3′ whose bases are complementary to a 20-base region, bases 260-280 from the beginning of the poly(A)[+] tail of λbTP3, is used. The double-stranded cDNA, synthesized by the method described previously (22,23), is tailed with dCTP, annealed to pBR322 that is dG-tailed at the PstI restriction site, and transformed into E. coli DH1 cells by the calcium chloride method (21).

The second cDNA library is screened with $^{32}$P-labeled oTP-1 (kaz2; ref. 6: specific activity: 0.5-1 x 10[9] cpm/μg DNA). Since the primer extension is carried out from the 5′ end of bTP3, and because pTP-1 is highly homologous to oTP-1, kaz2 (550 bases long from the 3' end of oTP-1) is used to identify positive clones that contain the 5' regions of bTP-1 cDNA. About 24,000 colonies (3,000 colonies 150-mm plate) are plated and replica filters are made according to a standard method (21). The filters are prehybridized at 42°C for 2 h in 5X SSC, 50 % (v/v) formamide, 0.6 % (w/v) SDS, 0.5 % (w/v) nonfat dry milk, 20 mM Tris HCl (pH 7.5), 4 mM EDTA and 0.5 mg/ml single-stranded herring sperm DNA and hybridized at 42°C for 18 h in the above buffer which contains the $^{32}$P-labeled oTP-1 cDNA (2 x $10^6$ cpm ml). The filters are washed twice for 15 min at 42°C with 5X SSC, 0.1 % (w/v) SDS and once for 15 min at 42°C with 2X SSC, 0.1 % (w v) SDS. Of about 200 positiv clones identified, plasmids with an inserted bTP-1 cDNA are isolated from 60 colonies (21). These plasmids are digested with PstI restriction endonuclease and subjected to agarose gel electrophoresis to determine the sizes of the bTP-1 cDNA's. The largest bTP-1 cDNA isolated from a pBR322 library is 677 bp in length (pbTP159). Sequencing is carried out as above (28,29).

## Screening λgtII Library with bTP-1 cDNA

Since the primer extension is carried out from a point 300 nucleotides from the poly(A)$^+$ tail, there is at this stage no full-length bTP-1 cDNA represented by a single clone in the pBR322 plasmid library. In order to obtain the full-length bTP-1 cDNA's from the λgtII library, a nick-translated probe is prepared from the 5'-end of pbTP159 (pbTP159-5': base positions 1-264) to rescreen the gtII library. About 7,500 recombinant phages are plated with E. coli Y1090 (2,500 plaques/plate) and kept at 37°C overnight. The plates are overlaid with duplicate facsimile filters and incubated with $^{32}$P-labeled pbTP159-5'. About 30 plaques are identified as positive clones containing bTP-1 cDNA. Phage DNA is isolated from 18 positive plaques, digested with EcoRI restriction endonuclease, and subjected to Southern hybridization (30) with a specific 3'-end probe ($^{32}$P-labeled λbTP3) to identify which of the bTP-1 cDNA's identified by pbTP159-5' also contains a region corresponding to the 3'-end of bTP-1 mRNA. Two-phage isolates (λbTP509 and λbTP517, 1035 and 950 bases long, respectively) are determined to have the 3' end of bTP-1 cDNA. Phage particles are prepared by the plate-lysate method and phage DNA's are purified by the method described by Reddy et al.) (31). DNA from λgtII clone is digested with EcoRI and the cDNA isnerts subcloned into the plasmid vector pUC13.

The nucleotide sequencing strategy for all three of the sequences reported in Fig. 2 is shown in Fig. 7.

## Western Blot Analysis of Fusion Proteins

E. coli Y1090 cells are infected with either wild type phage or λtII recombinants that contain the cDNA inserts λbTP3 or kaz2 (6) (an oTP-1 cDNA) at 37°C and plated on top agarose containing 10 mM isopropylthiogalactoside. Upon appearance of plaques, fifteen are picked from each plate, suspended together in SDS-sample buffer (32) and heated at 100°C for 5 min. The bacterial and phage proteins are separated by SDS-polyacrylamide gel electrophoresis (SDS-PAGE) on 7.5 % (w/v) gels (32) and electrophoretically transferred to nitrocellulose paper (33). Following transfer, the nitrocellulose filters are incubated with 5 % (w/v) non-fat dry milk ("blotto") in 10 mM sodium phosphate buffer, pH 7.2 containing 0.15M NaCl and 0.05 % (v/v) Tween 20 (PBST) for 2 H at 37°C. The blots are briefly rinsed with PBST and incubated with rabbit antiserum raised against oTP-1 (1:250) (27) for 2 h at 37°C. The filters are washed three time with PBST and the fusion proteins visualized with goat anti-rabbit IgG conjugated to horse radish peroxidase as described by Kazemi et al. (34).

## Expression of bTP-1 in Escherichia coli under control of pLac

A cDNA clone bTP-1A (Fig. 8) is derived from bTP-509 (shown in Fig. 2, but also represented in Figs. 5 and 6) by the ligation of:

i) A 36-base pair fragment (see Fig. 8) which is synthesized by annealing two complementary oligonucleotides that encoded an initiating methionine and amino acids 1-10 of the mature protein. This oligonucleotide possesses cohesive 5' (Cla I) and 3' (Ban I) termini.

ii) A 730-bp fragment of bTP-509 which is isolated by dual digestion with Ban I/Ssp I and which encodes amino acids 11-172 of the mature protein. It, therefore, possessed a 5' cohesive (Ban I) and 3' blunt (Ssp I) termini.

iii) The vector, pLac (Fig. 9), from which a region in the multicloning site of a synthetic Lac promoter is removed by dual digestion with Cla I Sma I (see Fig. 8).

E. coli strain JM-101 is transformed with the pBR322 plasmid that contains the above ligation products. Selection of transformants was made on Luria Broth Ampicillin plates.

The correct alignment of the ligated fragments is confirmed by restriction analysis of the plasmid DNA and then by complete sequencing of the entire insert.

The expression construct thus obtained is designated: pLac bTP-1A. Its expression is examined in E. coli (strains JM-101, JM-103, D-112, K-12) in Luria Broth supplemented with isopropyl thio-$\beta$-D-galactosidase (IPTG) (0.5, 1, 5 and 10mM) as the inducer of the Lac promoter (39). Controls minus inducer or in E. coli strains lacking the pLac bTP-1A vector are performed in parallel.

The results of this trial shows that bTP-1 is induced by IPTG but expression is relatively low (results not shown). Therefore, expression is attempted under the control of the pTrp 2 promoter.

## Expression of bTP-1 in Escherichia coli under the control of pTrp 2

i) The entire bTP-1A cDNA as shown in Fig. 8 is excised by cutting with the restriction endonucleases Cla I and Bam HI. These are ligated into the vector pTrp 2 which has been described in detail by Olsen et al. (40) and derives from the operator of the Trp operon of E. coli (41). This insertion is achieved by removing a Cla I Bam HI segment from the multicloning site on the vector (Fig. 10).

ii) E. coli strain DH-1 is transformed with the ligation products. Selection takes place on Luria Broth (LB)/Ampicillin plates which are supplemented with tryptophan (100 $\mu$g/ml) to repress expression. DNA in selected colonies is screened by restriction analysis. The integrity of the sequences is further confirmed by sequencing the entire DNA insert.

iii) Expression is examined in E. coli strains JM-103, D-112 and K-12 in M9 medium supplemented with alkali-hydrolyzed casein as a source of amino acids (M9/CA medium) in presence and absence of Trp and after addition of indole acetic acid (IAA; 1mM0 which acts as an inducer (41). Controls includ strains transfected with vector minus insert (pTrp 2). Cell growth is initiated at 37°C at a starting optical density of 0.02. Growth is terminated when the cell density reaches approximately 0.8 optical density units.

Bacteria are centrifuged, washed in medium and solubilized directly in SDS-electrophoresis buffer (32). Analysis is performed in 12.5 % polyacrylamide gels. Proteins are silver stained by standard procedures. High levels of expression are noted for bTP-1 (Fig. 11) in strain D-112. Densitometric scans (not shown) indicates that approximately 28 % of the total protein expressed in strain D-112 corresponds with a band of Mr 20,000 that appears to be induced specifically by absence of tryptophan or presence of IAA. Western blots (33) exposed to monoclonal antibody to the related ovine trophoblast protein-1 does not stain positively. However, when a polyclonal antibody prepared against a peptide for the terminal 20 amino acids of bTP-1 is used to stain the Western blots, a strong positive reaction is obtained (results not shown).

Therefore, expression of bTP-1 is achieved in E. coli strain D-112 at high levels under the control of the pTrp-2 promoter. This will allow production of large quantities of the bTP-1 protein necessary for enhancing fertility in mammals.

## Purification of recombinant bTP-1

E. coli D-112 carrying the pTrp-2/bTP-1A plasmid is cultured overnight in LB medium containing ampicillin and 0.1 mg/ml tryptophan. Next morning quantities of this stock culture are inoculated into one-liter volumes of fresh medium lacking tryptophan but containing 1mM IAA. The initial optical density of the culture is 0.02. The cultures are shaken rapidly at 37°C for 4 h, at which time the O.D. of the medium reaches 0.8. Cells are harvested by centrifugation, and the resulting cell pellet suspended in 20 ml of Tris-HCl buffer (0.01M, pH 8.0). The suspension is passed twice through a French Press (1500 psi). The broken cells are centrifuged (10,000 g; 30 min) to isolate particulate material. The loose membranous layer overlying the "hard" pellet is removed. The pellet is then resuspended in 0.01M Tris-HCl buffer containing 1mM EDTA and 1mM phenylmethane sulfonyl fluoride and the centrifugation step repeated.

The pellet of enriched inclusion bodies is dissolved in 25 ml of 4M guadidinium-HCl in 0.01M Tris-HCl buffer (pH 8.0) containing 0.25 % 2-mercaptoethanol by volume.

This solution is stirred overnight at 4°C. At this stage 25 ml of 0.02M Tris-HCl buffer is added drop by drop and the solution dialyzed against 4 changes (each 2 liter) of 0.02M Tris-HCl (pH 8.0) buffer over 16 h. The small amounts of protein that precipitate are collected by centrifugation. The supernatant solution contains predominantly solubilized, renatured bTP-1. To assess purity of the bTP-1, protein samples are analyzed by electrophoresis at each stage of the purification. Each fraction was also assessed for antiviral activity (Table 2).

The procedure provides a total of about 10 mg of highly purified bTP-1 from approximately 280 mg of starting protein. It is likely that yields can be improved by optimizing cell breakage and centrifugation procedures. The bTP-1 so produced has a specific activity of $4.1 \times 10^7$ laboratory units/mg which is quite similar to that of a recombinant bovine $IFN\text{-}\alpha_I\text{-}1$. It is also in the range of activities noted for other recombinant IFN of human and murine source (42). Despite the fact that this bTP-1 is not glycosylated, it appears to be relatively stable in solution, although experiments to characterize its properties are continuing. Surprisingly, the antiviral activity of the recombinant product is more than 10-fold higher than bTP-1 purified from conceptus culture medium (Table 1).

Thus, it is clearly proved possible to express bTP-1 in a bacterial host, for example in E. coli by employing the modified bTP-1 cDNA clone shown in Fig. 8. This cDNA is placed under the control of the pTrp-2 promoter. The resulting product is purified and shows the antiviral activity expected of a recombinant $IFN\text{-}\alpha$. E. coli (strain DH-1) containing the pTrp-2 bTP-1A expression plasmid has been deposited with the American Type Tissue Culture Collection.

## Table 2: Recovery of active bTP-1 from E. coli D-112

| Fraction | Cells* | Volume (ml) | Protein (mg) | Total Activity (U) | Specific Activity (U/mg) |
|---|---|---|---|---|---|
| Broken Cells[1] | Control | 27 | 158 | 0 | – |
|  | + Trp-2 | 29 | 282 | $2.1 \times 10^7$ | $7.4 \times 10^4$ |
| Supernatant[2] fraction | Control | 31 | 163 | 0 | – |
|  | + Trp-2 | 30 | 255 | $2.2 \times 10^7$ | $8.6 \times 10^4$ |
| Solubilized[3] inclusion bodies | Control | 35 | 4.2 | – | – |
|  | + Trp-2 | 50 | 24 | $1.5 \times 10^6$ | $6 \times 10^4$ |
| Renatured[4] protein | Control | 35 | 5.6 | – | – |
|  | + Trp-2 | 50 | 10 | $4.2 \times 10^8$ | $4.2 \times 10^7$ |
| Precipitated[5] protein | Control | 35 | 0.4 | – | – |
|  | + Trp-2 | 50 | 5 | $1.4 \times 10^6$ | $2.8 \times 10^5$ |

The units are laboratory units where 1 unit is equivalent to 8.8 International Reference Units (IRU) when compared to the human IFN- (Leukocyte) Reference Standard provided

by the National Institute of Allergy and Infectious Disease, Bethesda, MD. Thus, the specific activity of the final preparation would be approximately 3.0 x $10^8$ IRU/mg.

[1]After breakage suspension was assayed directly for antiviral activity (35,36).

[2]Supernatant remaining after centrifugation. Note that all of the active IFN in the cells was in the soluble fraction.

[3]Washed inclusion bodies had been solubilized in guanidium-HCl plus mercaptoethanol. Activity was relatively low, but some active protein was present.

[4]Solubilized protein in inclusion bodies after removal of guanidium-HCl and mercapto-ethanol by dialysis. Large amounts of active IFN are now detectable.

[5]This is the fraction which precipitated out after dialysis.

*Controls represent recoveries of protein and IFN activity from E. coli D-112 that did not contain the pTrp-2 expression plasmid. No IFN activity was present in broken cells and supernatant fraction. Activity was not followed during remaining protocol.

## REFERENCES

1. Bartol FF, Roberts RM, Bazer FW, Lewis GS, Godkin JD, Thatcher WW, (1985) "Characterization of proteins produced in vitro by periattachment bovine conceptuses", Biol Reprod 32:681-693.

2. Helmer SD, Hansen PJ, Anthony RV, Thatcher WW, Bazer FW, Roberts RM, (1987) "Identification of bovine trophoblast protein-1, a secretory protein immunologically related to ovine trophoblast protein-1", J Reprod Fert 79:83-91.

3. Knickerbocker JJ, Thatcher WW, Bazer FW, Barron DH, Roberts RM, (1986) "Inhibition of uterine prostaglandin F-2$\alpha$ production by bovine conceptus secretory proteins", Prostaglandins 31:777-793.

4. Bazer FW, Vallet JL, Roberts RM, Sharp DC, Thatcher WW, (1986) "Role of conceptus secretory products in establishment of pregnancy", J Reprod Fert 76:841-850.

5. Kozak M 1986 Point mutations define a sequence flanking the AUG initiator codon that modulates translation by eukaryotic ribosomes. Cell 44:283-292.

5. Kozak M, (1986) "Point mutations define a sequence flanking the AUG initiator codon that modulates translation by eukaryotic ribosomes", Cell 44.283-292.

6. Imakawa K, Anthony RV, Kazemi M, Marotti KR, Polites HG, Roberts RM, (1987) "Interferon-like sequence of ovine trophoblast protein secreted by embryonic trophectoderm", Nature 330:377-379.

7. von Heijne G, (1986) "A new method for predicting signal sequence cleavage sites", Nucleic Acids Res 14:4683-4690.

8. von Heijne G, (1985) "Signal sequences: the limits of variation", J Mol Biol 184:99-105.

9. Goeddel DV, Leung DW, Dull TJ, Gross M, Lawn RM, McCandliss R, Seeburg PH, Ulrick A, Yelverton E, Gray PW, (1981) "The structure of eight distinct cloned human leukocyte interferon cDNAs", Nature 290:20-26.

10. Lawn RM, Gross M, Houck CM, Franke AE, Gray PW, Goeddel DV, (1981) "DNA sequence of a major human leukocyte interferon gene", Proc Natl Acad Sci USA 78:5435-5439.

11. Capon DJ, Shepard HM, Goeddel DV, (1985) "Two distinct families of human and bovine

"interferon-α genes are coordinately expressed and encode functional polypeptides", Molec Cell Biol 5:768-779.

12. Shaw GD, Boll W, Taira H, Mantei N, Lengyel P, Weissmann C, (1981) "Structure and expression of cloned murine IFN-α genes", Nucleic Acid Res 11:555-573.

13. Dijkema R, Pouwels P, de-Reus A, Schellekens H, (1984) "Structure and expression in Escherichia coli of a cloned rat interferon-α gene", Nucleic Acids Res 12:1227-1243.

14. Lefevre F, LaBonnardiere C, (1986) "Molecular cloning and sequencing of a gene encoding biologically active porcine α-interferon", J Interferon Res 6:349-360.

15. Taniguchi T, Ohno S, Fujii-Kurimaya Y, Muramatsu M, (1980) "The nucleotide sequence of human fibroblast interferon cDNA", Gene 10:11-15.

16. Wetzel R, (1981) "Assignment of the disulfide bonds of leukocyte interferon", Nature 289:606-607.

17. Velan B, Koben S, Grosfeld H, Leiter M, Shafferman A, (1985) "Bovine interferon genes, structure and expression", J Biol Chem 260:5498-5504.

18. Pestka S, Lunger JA, Zoon KC, Samuel CE, (1987) "Interferons and their actions", Ann Rev Biochem 56:727-777.

19. Hansen PJ, Anthony RV, Bazer FW, Baumbach GA, Roberts RM, (1985) "In vitro synthesis and secretion of ovine trophoblast protein-1 during the period of maternal recognition of pregnancy", Endocrinology 117:1424-1430.

20. Anthony RV, Helmer SD, Sharif SF, Roberts RM, Hansen PJ, Thatcher WW, Bazer FW, (1988) "Synthesis and processing of ovine trophoblast protein-1 and bovine trophoblast protein-1, conceptus proteins involved in the maternal recognition of pregnancy", Endocrinology 123:1274-1280.

21. Maniatis T, Fritsch EF, Sambrook J, (1982) Molecular cloning. A laboratory manual. Cold Spring Harbor Laboratory, Cold Spring Harbor, NY.

22. Gubler U, Hoffman BJ, (1983) "A simple and very efficient method for generating cDNA libraries", Gene 25:263-269.

23. Polites HG, Marotti KR, (1986) "A step-wise protocol for cDNA synthesis", Biotechniques 4:514-520.

24. Huyhn TV, Young RA, Davis RW, (1985) "Constructing and screening cDNA libraries in λgt10 and λgt11", In: Glover DM (ed), DNA cloning 1: A Practical Approach. IRL Press, Washington DC, pp 49-78.

25. Young RA, Davis RW, (1983) "Efficient isolation of genes by using antibody probes", Proc Natl Acad Sci USA 80:1193-1198.

26. Godkin JD, Bazer FW, Moffatt J, Sessions F, Roberts RM, (1982) "Purification and properties of a major, low molecular weight protein released by the trophoblast of sheep blastocysts on day 13-21", J Reprod Fert 65:141-150.

27. Godkin JD, Bazer FW, Roberts RM, (1984) "Ovine trophoblast protein-1 (oTP-1), an early secreted blastocyst protein, binds specifically to uterine endometrium and affects protein synthesis", Endocrinology 114:120-130.

28. Chen E, Seeburg PH, (1985) "Supercoil sequencing: a fast and simple method for sequencing plasmid DNA", DNA 4:165-170.

29. Sanger F, Nicklen S, Coulson AR, (1977) "DNA sequencing with chain-terminating inhibitors", Proc Natl Acad Sci USA 74:5463-5467.

30. Southern E, (1975) "Detection of specific sequences among DNA fragments separated by gel electrophoresis", J Mol Biol 98:503-517.

31. Reddy KJ, Kuwabara T, Sherman LA, (1988) "A simple and efficient procedure for the isolation of high-quality phage DNA using a DEAE-cellulose column", Analyt Biochem 168:324-331.

32. Laemmli UK, (1976) "Cleavage of structural proteins during the assembly of the head of bacteriophage T₄", Nature 227:680-685.

33. Towbin H, Staehelin T, Gordon J, (1979) "Electrophortransfer of proteins from polyacrylamide gels to nitrocellulose sheets: Procedures and some applications", Proc Natl Acad Sci USA 76:4350-4354.

34. Kazemi M, Malathy PV, Keisler DH, Roberts RM, (1988) "Ovine trophoblast protein-1 and bovine trophoblast protein-1 are present as specific components of uterine flushin of pregnant ewes and cows", Biol Reprod, 39:457-463.

35. Brennan GL, Kronenberg LH, (1983) "Automated bioassay of interferons in microtest plates", Biotechniques 1:78-83.

36. Roberts RM, Imakawa K, Niwano Y, Kazemi M, Malathy PV, Hansen TR, Glass AA, Kronenberg LH, (1989) "Interferon production by the preimplantation sheep embryo", J Interferon Res, 9:175-187.

37. Caput D, Bentler B, Hortog K, Thayer R, Brown-Shimer S, Cerami A, (1986) "Identification of a common nucleotide sequence in the 3'-intranslated region of mRNA molecules specifying inflammatory mediators", Proc Natl Acad Sci USA 83:1670-1674.

38. Anthony RV, Helmer SD, Sharif SF, Roberts RM, Hansen PJ, Thatcher WW, Bazer FW, (1988) "Synthesis and processing of ovine trophoblast protein-1 and bovine trophoblast protein-1 conceptus secretory proteins involved in the maternal recognition of pregnancy", Endocrinol 123:1274-1280.

39. Reznikoff, W.S., Abelson, J.N. (1978) The lac promoter. In: The Operon (Miller, J.H. and Reznikoff, W.S. eds) Cold Spring Harbor Laboratory, New York, pp 221-243.

40. Olsen, M.K. Rockenbach, S.K., Curry, K.A., Tomich, C-S.C. (1989) Enhancement of heterologous polypeptide expression by alterations in the ribosome-binding site sequence. J. Biotech. 9:179-190.

41. Platt, T. (1978) Regulation of gene expression in the tryptophan operon of Escherichia coli. In: The Operon (Miller, J.H. and Reznikoff, W.S. eds) Cold Spring Harbor Laboratory, New York, pp 263-302.

42. Pestka, S., Langer, J.A., Zoon, K.C. and Samuel, C.E. (1987) Interferons and their actions. Ann. Rev. Biochem. 56:727-777.

## Claims

1. A recombinant DNA sequence capable of expressing a bovine trophoblast protein-1 in a suitable host cell.

2. A DNA sequence according to claim 1 capable of expressing the polypeptide shown in Figure 1 or a functional variation thereof.

3. A DNA sequence according to claim 2 having the formula shown in Figure 1.

4. A recombinant DNA sequence encoding a bovine trophoblast portein-1 or a functional variation thereof.

5. A recombinant DNA sequence according to claim 4 operably linked with regulatory sequences effecting the expression of a bovine trophoblast protein-1 in a suitable host cell.

6. A recombinant vector adapted for transformation of a suitable host cell, said vector comprising a DNA sequence capable of expressing a bovine trophoblast protei-1 in said host cell.

7. A recombinant vector according to claim 6 consisting of a plasmid adapted for the transformation of a bacterial cell.

8. A recombinant vector according to claim 6 adapted for the transformation of a mammalian cell.

9. A recombinant vector according to claim 6 adapted for the transformation of a yeast cell.

10. A process of preparing bovien trophoblast protein-1 which comprises the steps of:

    (a) introducing a recombinant vector comprising the DNA sequence of claim 1 into a suitable host cell,

    (b) culturing the resulting transformed host cell in a suitable culturing medium, and

    (c) isolating the recombinant bovine trophoblast protein-1 from the cultured cells.

11. Essentially pure bovine trophoblast protein-1 without the contaminations of bovine trophoblast protein-1 isolated from natural sources..

12. Bovine trophoblast protein-1 according to claim 11 prepared by means of recombinant DNA technology.

13. Bovine trophoblast protein-1 according to claim 12 produced by a bacterium.

14. Recombinant protein of the bovine trophoblast protein-1 type according to claim 12 having substantially the mammalian fertility enhancing properties of the naturally occuring bovine trophoblast protein-1.

15. A method for enhancing fertility in mammals comprising administering to said mammal an effective mammalian fertility enhancing amount of a bovine trophoblast protein-1.

16. A method according to claim 15 wherein the bovine trophoblast protein-1 is a recombinant protein.

17. A method according to claim 15 wherein the bovine trophoblast protein-1 is administered in a daily dosage of 0.5 to 50 mg/animal.

18. A pharmaceutical composition for enhancing fertility in mammal comprising an effective mammalian fertility enhancing amount of bovine trophoblast protein-1 or a protein having substantially the mammalian fertility enhancing activity of bovine trophoblast protein-1 together with non-toxic carriers or adjuvants.

bTP-1 . GATCCCCGGCAAACTAGAATTCACCTGAAGGTTCACCCAGACCCCATCTCAGCCAGCCCAGCAGCAGCCACATCTTCCCC

s1                                                                          s23  1  2
met ala phe val leu ser leu leu met ala leu val leu val ser tyr gly gln gly arg ser leu gly cys tyr
ATG GCC TTC GTG CTC TCT CTA CTG ATG GCC CTG GTG CTG GTC AGC TAC GGC CAG GGA CGA TCT CTG GGT TGT TAC

3                                                                        27
leu ser glu asp his met leu gly ala arg glu asn leu arg leu leu ala arg met asn arg leu ser pro his
CTG TCT GAG GAC CAC ATG CTA GGT GCC AGG GAG AAC CTC AGG CTC CTG GCC CGA ATG AAC AGA CTC TCT CCT CAT

28                                                                    52
pro cys leu gln asp arg lys asp phe gly leu pro gln glu met val glu gly asn gln leu gln lys asp gln
CCC TGT CTG CAG GAC AGA AAA GAC TTT GGT CTT CCT CAG GAG ATG GTG GAG GGC AAC CAG CTC CAG AAG GAT CAG

53                                                                        77
ala ile ser val leu his glu met leu gln gln cys leu asn leu phe tyr thr glu his ser ser ala ala trp
GCT ATC TCT GTG CTC CAC GAG ATG CTC CAG CAG TGC CTC AAC CTC TTC TAC ACA GAG CAC TCG TCT GCT GCC TGG

78                                                                  102
asn thr thr leu leu glu gln leu cys thr gly leu gln gln gln leu glu asp leu asp ala cys leu gly pro
AAC ACC ACC CTC CTG GAG CAG CTC TGC ACT GGG CTC CAA CAG CAG CTG GAG GAC CTG GAC GCC TGC CTG GGC CCA

103                                                           127
val met gly glu lys asp ser asp met gly arg met gly pro ile leu thr val lys lys tyr phe gln gly ile
GTG ATG GGA GAG AAA GAC TCT GAC ATG GGA AGG ATG GGC CCC ATT CTG ACT GTG AAG AAG TAC TTC CAG GGT ATC

128                                                           152
his val tyr leu lys glu lys glu tyr ser asp cys ala trp glu ile ile arg val glu met met arg ala leu
CAT GTC TAC CTG AAA GAA AAA GAA TAC AGT GAC TGC GCC TGG GAA ATC ATC AGA GTG GAG ATG ATG AGA GCC CTC

153                                                  172
ser ser ser thr thr leu gln lys arg leu arg lys met gly gly asp leu asn ser leu end
TCT TCA TCA ACC ACC TTG CAA AAA AGG TTA AGA AAG ATG GGT GGA GAT CTG AAC TCA CTT TGA GATGACTCTCGCTGAC

AAGTATGCCACATCACCTTCGTACACTCACCTGTGTTCATTTCAGAACACTCTCATTTCTGCTTCAGCCACCCAAATCATTGAATTACTTTAAGTGATAC

TTTGTCAGCAGTAATAAGCAAGTAGATATAAAAGTACTCAGCTGTAGGGGCATGAGTCCTTAAGTCATGCCTGCCCTGATGTTATCTGTTGTTGATTTAT

GTATTCCTTCTTGCATCTAACATACTTAAAATATTAGGAAATTTGTAAAGTTACATTTCATTTGTACATCTATTAAAAATTTCTAAAACATGTTTACCATT

TTGTGTTATTAAATTTGTCCTTTGTTCTATTTATTAAATCAAAGAAAATGAAAAAAAAAAAAAAAAAAAAAA

# FIG.1

EP 0 367 063 A1

```
        *  ***    *** ***        * *
     GGTCTAGGG  GTGAACCTTCA  GGTAGGTTCTCCTAGACCCCATCTCAGCAAGCCCAGCAGCAGCCACATCTTCCCCATGGCCTTCGTGCTCTCTCTA
1    CATCCCCGCAAACTACAATTCACCTGAAGGTTCACCCACACCCCATCTCAGCCAGCCCAGCAGCAGCCACATCTTCCCCATGGCCTTCGTGCTCTCTCTA
                            AGCAGCAGCCACATCTTCCCCATGGCCTTCGTGCTCTCTCTA

                              *     *    ***                *
     CTGATGGCCCTGGTGCTGGTCAGCTATGGCCCGGGAGAGTCTCTGGGTTGTTACCTGTCTGAGAACCACATGCTAGGTGCCCAGGGAGAACCTCAGGCTCC
101  CTGATGGCCCTGGTGCTGGTCAGCTACGGCCCAGGGACCATCTCTGGGTTGTTACCTGTCTGAGGACCACATGCTAGGTGCCCAGGGAGAACCTCAGGCTCC
     CTGATGGCCCTGGTGCTGGTCAGCTACGGCCCGGGACCATCTCTGGGTTGTTACCTGTCTGAGGACCACATGCTAGGTGCCCAGGGAGAACCTCAGGCTCC
                               *

     TGGCCCGAATGAACAGACTCTCTCCTCATCCCTGTCTGCAGGACAGAAAAGACTTTGGTCTTCCTCAGGACATGGTGGAGGGCAACCAGCTCCAGAAGGA
201  TGGCCCCGAATGAACACACTCTCTCCTCATCCCTGTCTGCAGGACAGAAAAGACTTTGGTCTTCCTCAGGACATGGTGGAGGGCAACCAGCTCCAGAAGGA
     TGGCCCCGAATGAACAGACTCTCTCCTCATCCCTGTCTGCAGGACAGAAAAGACTTTGGTCTTCCTCAGGACATGGTGGAGGGCAACCAACTCCAGAAGGA
                                                                                               *

                     *              *                                      .
     TCAGGCTATCTCTGTGCTCCATGAGATGCTCCAGCAGTGCCTTCAACCTCTTCTACACAGAGCACTCGTCTGCTGCCCTGGAACACCACCCTCCTGGAGCAG
301  TCAGGCTATCTCTGTGCTCCACGAGATGCTCCAGCAGTGCCTCAACCTCTTCTACACAGAGCACTCGTCTGCTGCCCTGGAACACCACCCTCCTGGACCAG
     TCAGGCTATCTCTTTGCTCCATGAGATGCTCCAGCAGTGCTTTAACCTCTTCTACACAGAGCACTCGTCTGCTGCCCTGGAACACCACCCTCCTGGAGCAG
               *        *                  * *

     CTCTGCACTGGGCTCCAACAGCAGCTGGAGGACCTGGACGCCCTGCCTGGGCCCAGTCATGGGAGAGAAAGACTCTGACATGGGAAGGATGGGCCCCATTC
401  CTCTGCACTGGGCTCCAACAGCAGCTGGAGGACCTGGACGCCCTGCCTGGGCCCAGTCATGGGAGAGAAAGACTCTGACATGGGAAGGATGGGCCCCATTC
     CTCTGCACTGGGCTCCAACAGCAGCTGGAGGACCTGGACGCCCTGCCTGGGCCCAGTCATGGGAGAGAAAGACTCTGACATGGGAAGGATGGGCCCCATTC

                    *                                               *
     TGACTGTGAAGAAGTACTTCCAGGGCCATCCATGTCTACCTGAAAGAAAAAGAATACAGTGACTGCCGCCCTGGGAAATCATCAGAATGGAGATGATGACAGC
501  TCACTGTGAAGAAGTACTTCCAGGGTATCCATGTCTACCTGAAAGAAAAAGAATACAGTGACTGCCGCCCTGGGAAATCATCAGAGTGGAGATCATGACAGC
     TGACTGTGAAGAAGTACTTCCAGGGCATCCATGTCTACCTGAAAGAAAAAGAATACAGTGACTGCCGCCCTGGGAAATCATCAGAATGGAGATGATGACAGC
                    *                                               *

                                                                              *
     CCTCTCTTCATCAACCACCTTGCAAAAAAAGGTTAACAAAGATGGGTGGACATCTGAACTCACTTTGACATGACTCTCGCTGACTAAGATGCCACATCAGC
601  CCTCTCTTCATCAACCACCTTGCAAAAAAAGGTTAAGAAAGATGGGTGGACATCTGAACTCACTTTGAGATGACTCTCGCTGACTAAGATGCCACATCACC
     CCTCTCTTCATCAACCACCTTGCAAAAAAAGGTTAAGAAAGATGGGTGGACATCTGAACTCACTTTGACATGACTCTCGCTGACTAAGATGCCACATCACC

     *  *    * *                                    *     *          **         * *
     CTCCTACACCCGCCTGTGTTCATTTCAGAACACTCTCGATTTCTGCTCCAGCCACCAAATTCATTGAATTACTTTAGGTGATACTTTGTCAGTACTAAAAA
701  TTCGTACACTCACCTGTGTTCATTTCAGAACACTCTCGATTTCTGCTTCAGCCACCGAAATCATTGAATTACTTTAACTGATACTTTGTCAGCAGTAATAA
     TTCGTACACTCACCTGTGTTCATTTCAGAACACTCTCGATTTCTGCTTCAGCCACCG     ATTCAATTACTTTAACTGATACTTTGTCACCAGCAATAA
                                                                                            *

               *                *  *     *                   *         * *        *    *
     GCAAGTAGATATAAAAGTATTCAGCTGTAGGGGCATCAGTCCTGAAATGATGCCTTCCCTGATGTTATCTGTTGCTGATTTATTTATACCTTCTAGCATT
801  GCAAGTAGATATAAAAGTACTCAGCTGTAGGGGCATCAGTCCTTAAGTGATGCCTGCCCTGATGTTATCTGTTGTTGCATTTATGTATTCCTTCTTGCATC
     GCAAGTAGATATAAAAGTACTCAGCTGTAGGGGCATAAGTCCTTAAGTGATGCCTGCCCTGATGTTATCTGTTGTTGCATTTATGTATTCCTTCTTGCATC
                                           *

                      *                *
     TAACATACTTAAAATATTAGGCAAATTTGTTAAGTTACATTTACATCTGTACATCATATTAAAATTTCTAAAACAAAAA
901  TAACATACTTAAAATATTAGGCAAATTTGTAAAGTTACATTT CATTTGTACATC TATTAAAATTTCTAAAACATGTTTACCATTTTGTGTTATTAAATT
     TAACATACTTAAAATGTTAGGCAAATTTGTAAAGTTACATTT CATTTGTACATC TATTAAAATTTCTAAAACATGTTTAAAAAAAAAAAAAAAAAAAAAA
               *

1001 TGTCCTTTGTTCTATTTATTAAATCAAAGAAAATGAAAAAAAAAAAAAAAAA
     AAA
```

FIG.2

FIG.3

FIG.4

```
bTP-1    GATCCCCGCAAAC TAGAATTCACCTGAAGGTTCACCCAGACCCCATCTCAGCCAGCCCAGCAGCAGCCACATCTTCCCC
oTP-1    CTCAGATGGGATCACAGAACCTACCTGAAGGTTCCCCCTGACCCCATCTCAGCCACCCCAGCAGCAGCCGCATCTTCCCC
         ****** * * **    ***            *    *                              *
```

```
s1                                                              s23   1   2
met ala phe val leu ser leu leu met ala leu val leu val ser tyr gly gln gly arg ser leu gly cys tyr
ATG GCC TTC GTG CTC TCT CTA CTG ATG GCC CTG GTG CTG GTC AGC TAC GGC CAG GCA CGA TCT CTG GGT TGT TAC
ATG GCC TTC GTG CTC TCT CTA CTG ATG GCC CTG GTG CTG GTC AGC TAT GGC CCA GGA GGA TCT CTG GGT TGT TAC
                                                            *       pro     gly
```

```
3                                                                                    27
leu ser glu asp his met leu gly ala arg glu asn leu arg leu leu ala arg met asn arg leu ser pro his
CTG TCT GAG GAC CAC ATG CTA GGT GCC AGG GAG AAC CTC AGG CTC CTG GCC CGA ATG AAC AGA CTC TCT CCT CAT
CTA TCT CGG AAA CTC ATG CTG GAT GCC AGG GAG AAC CTC AAG CTC CTG GAC CGA ATG AAC AGA CTC TCC CCT CAT
  *     arg lys leu     * asp                 lys         asp                         ser
```

```
28                                                                                   52
pro cys leu gln asp arg lys asp phe gly leu pro gln glu met val glu gly asn gln leu gln lys asp gln
CCC TGT CTG CAG GAC AGA AAA GAC TTT GGT CTT CCT CAG GAG ATG GTG GAG GGC AAC CAG CTC CAG AAG GAT CAG
TCC TGT CTG CAG GAC AGA AAA GAC TTT GGT CTT CCC CAG GAG ATG GTG GAG GGC GAC CAG CTC CAG AAG GAC CAG
ser                                         *                           asp                 *
```

```
53                                                                                   77
ala ile ser val leu his glu met leu gln gln cys leu asn leu phe tyr thr glu his ser ser ala ala trp
GCT ATC TCT GTG CTC CAC GAG ATG CTC CAG CAG TGC CTC AAC CTC TTC TAC ACA GAG CAC TCG TCT GCT GCC TGG
GCC TTC CCT GTG CTC TAC GAG ATG CTC CAG CAG AGC TTC AAC CTC TTC TAC ACA GAG CAC TCC TCT GCT GCC TGG
  * phe pro         tyr                 ser phe                         *
```

```
78                                                                                   102
asn thr thr leu leu glu gln leu cys thr gly leu gln gln gln leu glu asp leu asp ala cys leu gly pro
AAC ACC ACC CTC CTG GAG CAG CTC TGC ACT GGG CTC CAA CAG CAG CTG GAG GAC CTG GAC GCC TGC CTG GGC CCA
GAC ACC ACC CTC CTG GAG CAG CTC TGC ACT GGA CTC CAA CAG CAG CTG GAC CAC CTG GAC ACC TCC AGG GGT CAA
asp                                                         asp his         thr     arg * gln
```

```
103                                                                                  127
val met gly glu lys asp ser asp met gly arg met gly pro ile leu thr val lys lys tyr phe gln gly ile
GTG ATG GGA GAG AAA GAC TCT GAC ATG GGA AGG ATG GGC CCC ATT CTG ACT GTG AAG AAG TAC TTC CAG GGT ATC
GTG ATG GGA GAG GAA GAC TCT GAA CTG GGT AAC ATG GAC CCC ATT GTG ACC GTG AAG AAG TAC TTC CAG GGC ATC
                glu         glu leu * asn     asp         val *                         *
```

```
128                                                                                  152
his val tyr leu lys glu lys glu tyr ser asp cys ala trp glu ile ile arg val glu met met arg ala leu
CAT GTC TAC CTG AAA GAA AAG GAA TAC AGT GAC TGC GCC TGG GAA ATC ATC AGA GTG GAG ATG ATG AGA GCC CTC
TAT GAC TAC CTG CAA GAG AAG GGA TAC AGC GAC TGC GCC TGG GAA ATC GTC AGA GTC GAG ATG ATG AGA GCC CTC
tyr asp         gln * * gly         *                         val
```

```
153                                                 172
ser ser ser thr thr leu gln lys arg leu arg lys met gly gly asp leu asn ser leu end
TCT TCA TCA ACC ACC TTG CAA AAA AGG TTA AGA AAG ATG GGT GGA CAT CTG AAC TCA CTT TGA GATCACTCTCCCTCAC
ACT GTA TCA ACC ACC TTG CAA AAA AGG TTA ACA AAG ATG GGT GGA GAT CTG AAC TCA CCT TGA  TGACTCTTGCCCGAC
thr val                             thr                           pro end **    * *
```

```
AAGTATGCCACATCACCTTCGTACACTCACCTGTGTTCATTTCAGAAGACTCTGATTTCTGCTTCAGCCACCGAAATCATTGAATTACTTTAAGTGATAC
AAGTATGCCACATCAGCCTCCTACACCCGCCTGTGTTCATTTCAGAAGACTCTGATTTCTGCTTCAGCCACCAAATTCATTGAATTACTTTACCTGATAC
               * * *     * *                                *     * *             **
```

```
TTTGTCAGCAGTAATAAGCAAGTAGATATAAAAGTACTCAGCTGTAGGGGCATGAGTCCTTAAGTGATGCCTGCCCTGATGTTATCTGTTGTTGATTTAT
TTTGTCAGTAGTAAAAAGCAAGTAGATATAAAAGTATTCAGCTGTAGGGGCATGAGTCCTGAAATGATGCCTTCCCTGATGTTATCTGTTGCTGATTTAT
        *    *            *                         * *         *                     *
```

```
GTATTCCTTCTTGCATCTAACATACTTAAAAATATTAGGAAATTTGTAAAGTTACATTT CATTTGTACATC TATTAAAATTTCTAAAACATGTTTACCA
TTATACCTTCTAGCATTTAACATACTTAAAAATATTAGGAAATTTGTTAAGTTACATTTACATCTGTACATCATATTAAAATTTCTAAAACAAAAAAAAAA
* *     * *                           *             * *      *
```

```
TTTTGTGTTATTAAATTTGTCCTTTGTTCTATTTATTAAATCAAAGAAAATGAAAAAAAAAAAAAAAAAAAAA    FIG.5
```

```
bTP-1      GATCCCCGGAAACTAGAATTCACCTCAAGGTTCACCCAGACCCCATCTCAGCCAGCCCAGCAGCAGCCACATCTTCCCC
BoIFN- II      CAGAGAACCTACCTCAAGGTTCCACCAGACGCTGTCTCAGCCAGCCCAGCAGCAGCCTCATCTTCCCC
               ***    ***    *     **     * **                           *

                                                                       s23   1    2
s1
met ala phe val leu ser leu leu met ala leu val leu val ser tyr gly gln gly arg ser leu gly cys tyr
ATG GCC TTC GTG CTC TCT CTA CTG ATG GCC CTG GTG CTG GTC AGC TAC GGC CAG GGA CGA TCT CTG GGT TGT TAC
ATG GCC TTC GTG CTC TCT CTA CTG ATG GCC CTG GTG CTG GTC AGC TAT GGC CCG GGA GGA TCC CTG GGC TGT GAC
                                                        *   pro     gly  *       *    asp

                                                                                        27
3
leu ser glu asp his met leu gly ala arg glu asn leu arg leu leu ala arg met asn arg leu ser pro his
CTC TCT GAG GAC CAC ATG CTA GGT GCC AGG GAG AAC CTC AGG CTC CTG GCC CGA ATG AAC ACA CTC TCT CCT CAT
TTG TCT CCG AAC CAC GTG CTG GTT GGC AGG CAG AAC CTC AGG CTC CTG GGC CAA ATG AGG AGA CTC TCC CCT CGC
*       pro asn     val  * val gly     gln                 gly gln     arg         *    arg

28                                                                                      52
pro cys leu gln asp arg lys asp phe gly leu pro gln glu met val glu gly asn gln leu gln lys asp gln
CCC TGT CTG CAG GAC AGA AAA GAC TTT GGT CTT CCT CAG GAG ATG GTG GAG GGC AAC CAG CTC CAG AAG GAT CAG
TTC TGT CTG CAG GAC AGA AAA GAC TTT GCT TTC CCC CAG GAG ATG GTG GAG GTC ACC CAG TTC CAG GAG GCC CAG
phe                           ala phe  *               val ser     phe     glu ala

53                                                                                      77
ala ile ser val leu his glu met leu gln gln cys leu asn leu phe tyr thr glu his ser ser ala ala trp
CCT ATC TCT GTG CTC CAC GAG ATG CTC CAG CAG TGC CTC AAC CTC TTC TAC ACA GAG CAC TCG TCT CCT GCC TGG
GCC ATT TCT GTG CTC CAT GAG ATG CTC CAG CAG AGC TTC AAC CTC TTC CAC AAA GAG CGC TCC TCT CCT GCC TGG
 *    *               *                   ser phe               his lys     arg  *

78                                                                                      102
asn thr thr leu leu glu gln leu cys thr gly leu gln gln gln leu glu asp leu asp ala cys leu gly pro
AAC ACC ACC CTC CTG GAG CAG CTC TGC ACT GGG CTC CAA CAG CAG CTG GAG GAC CTG GAC GCC TGC CTG GGC CCA
GAC ACT ACC CTC CTG GAG CAG CTC CTC ACT GGA CTC CAT CAG CAG CTG GAT GAC CTG GAT GCC TGT CTG GGC CTG
asp   *                        leu   *     his               asp       *         *         leu

103                                                                                     127
val met gly glu lys asp ser asp met gly arg met gly pro ile leu thr val lys lys tyr phe gln gly ile
GTG ATG GGA GAG AAA GAC TCT GAC ATG GGA AGG ATG GGC CCC ATT CTG ACT GTG AAG AAG TAC TTC CAG GGT ATC
TTG ACT GGA GAG GAA GAC TCT GCC CTG GGA AGG ACG GGC CCC ACA CTG GCC ATG AAG AGG TAC TTC CAG GGC ATC
leu thr         glu         ala leu         thr         thr     ala met     arg             *

128                                                                                     152
his val tyr leu lys glu lys glu tyr ser asp cys ala trp glu ile ile arg val glu met met arg ala leu
CAT GTC TAC CTG AAA GAA AAA GAA TAC AGT GAC TGC GCC TGG GAA ATC ATC AGA GTG GAG ATG ATG AGA GCC CTC
CAT GTC TAC CTG CAA GAG AAG GGA TAC AGC GAC TGT GCC TGG GAA ATC GTC AGA CTG GAA ATC ATG AGA TCC TTG
             gln  *  * gly     *       *             val     leu * ile         ser * *

153                                                             172
ser ser ser thr thr leu gln lys arg leu arg lys met gly gly asp leu asn ser leu end
TCT TCA TCA ACC ACC TTG CAA AAA ACG TTA AGA AAG ATG GGT GGA GAT CTG AAC TCA CTT TGA GATCACTCTCGCTCACT
TCT TCA TCA ACC AGC TTG CAA GAA AGG TTA AGA ATG ATG GAT GGA GAC CTG AAA TCA CCT TGA CATGACTCTCACTGACT
             ser         glu             met     asp  *     lys     pro end *        *


AAGATGCCACATCACCTTCGTACACTCACCTGTGTTCATTTCAGAAGACTCTGATTTCTGCTTCAGCCACCGAAATCATTGAATTACTTTAACTGATACT
AAGATGCCCCATCATCTTTGCACACTCATCTGTGGCCATTTCAAAAGACTCTGATTTCTGTTGTACCCACAAAATTTATTGAATTACTTCAGCCAATACT
        *  *  * *       *    *       **         *              * **      ** * *         * * **


TTCTCAGCAGTAATAAGCAAGTACATATAAAAGTACTCAGCTGTAGGGGCATGAGTCCTTAAGTGATCCCTGCCCTCATGTTATCTGTTGTTGATTTATG
TTGTCAGTAGTA AATGAA TATACATAAATTTTTTGGCTGCAGGTGCATCAGTCCCGAAGTGAAGACTGCCCTTATTTTATTGTTTGCTTATTTAT
        *   ** ** * *       ** ** **     *   *    *     **      * *        * *    *** * *          *


TATTCCTTCTTGCATCTAACATACTTAAAATATTAGGAAATTTGTAAAGTTACATTTCATTTGTACA TCTATTAAAATTTCTAAAAGATGTTTACCATT
TTTGTTATATTTATTCTTTTAT TTCCTCATATT  TATTTTTCCATATAAAATATTTTTGTTTACATTGTATTAAAATTTAACAAATACATTAACATTT
 * ****  *  ***   *** **  ****      *** **   **** *** *** ** * *      * *                 *** * ** * **


TTGTGTTATTAAATTTGTCCTTTGTTCTATTTATTAAATCAAACAAAATGAAAAAAAAAAAAAAAAAAAA
TTATTTCATTATATTTGTAATTTGTTTTTATTTATTAAAT ATTGTCAAGGTGAACTTCTTGAATT
 * * *   *      **      *               * ** ** **
```

FIG.6

FIG.7

EP 0 367 063 A1

Subcloning of bTP to generate a clone (bTP-1A; from bTP-509) for the expresion of the mature protein

bTP-1                CATCCCCCGAAACTAGAATTCACCTGAAGGTTCACCCAGACCCCATCTCAGCCAGCCCAGCAGCAGCCACATCTTCCCC

```
                                                                s23   1    2
s1
met ala phe val leu ser leu leu met ala leu val leu val ser tyr gly gln gly arg ser leu gly cys tyr
ATG GCC TTC GTG CTC TCT CTA CTG ATG GCC CTG GTG CTG GTC AGC TAC GGC CAG GGA CGA TCT CTG GGT TGT TAC
                            bTP-1A:                                ----vector-----TA/CGATA ATG ... ...
                                                                                  Cla I   met

3                                                                                              27
leu ser glu asp his met leu  gly ala arg glu asn leu arg leu leu ala arg met asn arg leu ser pro his
CTG TCT CAG CAC CAC ATG CTA C GT CCC AGG GAG AAC CTC AGG CTC CTG GCC CGA ATG AAC ACA CTC TCT CCT CAT
... ... ... ... ... ... ... ./.. ... ... ... ... ... ... ... ... ... ... ... ... ... ... ... ... ...
                            Ban I

28                                                                                             52
pro cys leu gln asp arg lys asp phe gly leu pro gln glu met val glu gly asn gln leu gln lys asp gln
CCC TGT CTG CAG GAC AGA AAA GAC TTT GGT CTT CCT CAG GAG ATG GTG GAG GGC AAC CAG CTC CAG AAG GAT CAG
... ... ... ... ... ... ... ... ... ... ... ... ... ... ... ... ... ... ... ... ... ... ... ...

53                                                                                             77
ala ile ser val leu his glu met leu gln gln cys leu asn leu phe tyr thr glu his ser ser ala ala trp
GCT ATC TCT GTG CTC CAC GAG ATG CTC CAG CAG TGC CTC AAC CTC TTC TAC ACA GAG CAC TCG TCT GCT GCC TGG
... ... ... ... ... ... ... ... ... ... ... ... ... ... ... ... ... ... ... ... ... ... ... ...

78                                                                                             102
asn thr thr leu leu glu gln leu cys thr gly leu gln gln gln leu glu asp leu asp ala cys leu gly pro
AAC ACC ACC CTC CTG GAG CAG CTC TGC ACT GGG CTC CAA CAG CAG CTG GAG GAC CTG GAC GCC TGC CTG GGC CCA
*** ... ... ... ... ... ... ... ... ... ... ... ... ... ... ... ... ... ... ... ... ... ... ...

103                                                                                            127
val met gly glu lys asp ser asp met gly arg met gly pro ile leu thr val lys lys tyr phe gln gly ile
GTG ATG GGA GAG AAA GAC TCT GAC ATG GGA AGG ATG GGC CCC ATT CTG ACT GTG AAG AAG TAC TTC CAG GGT ATC
... ... ... ... ... ... ... ... ... ... ... ... ... ... ... ... ... ... ... ... ... ... ... ...

128                                                                                            152
his val tyr leu lys glu lys glu tyr ser asp cys ala trp glu ile ile arg val glu met met arg ala leu
CAT GTC TAC CTG AAA GAA AAA GAA TAC AGT GAC TGC CCC TGG GAA ATC ATC AGA GTG GAG ATG ATG AGA GCC CTC
... ... ... ... ... ... ... ... ... ... ... ... ... ... ... ... ... ... ... ... ... ... ... ...

153                                                               172
ser ser ser thr thr leu gln lys arg leu arg lys met gly gly asp leu asn ser leu end
TCT TCA TCA ACC ACC TTG CAA AAA AGG TTA AGA AAG ATG GGT GGA GAT CTG AAC TCA CTT TGA GATGACTCTCGCTGAC
... ... ... ... ... ... ... ... ... ... ... ... ... ... ... ... ... ... ... ... ... ..............
```

TAAGATGCCACATCACCTTCGTACACTCACCTGTGTTCATTTCAGAAGACTCTGATTTCTGCTTCAGCCACCGAAATCATTGAATTACTTTAACTGATAC
....................................................................................................

TTTGTCAGCAGTAATAAGCAAGTAGATATAAAAGTACTCAGCTGTAGGGGCATGAGTCCTTAAGTGATGCCTGCCCTGATGTTATCTGTTGTTGATTTAT
....................................................................................................

GTATTCCTTCTTGCATCTAACATACTTAAAAT ATTACGAAATTTGTAAAGTTACATTTCATTTGTACATCTATTAAAATTTCTAAAACATGTTTACCATT
................................/CGGTAAGTAACTAAGCATCC-----vector-----
                                  Ssp I             Bam HI

TTGTGTTATTAAATTTGTCCTTTGTTCTATTTATTAAATCAAACAAAATGAAAAAAAAAAAAAAAAAAAA

# FIG.8

Oligonucleotides for 5' portion of construct:

```
                0  1   2   3   4   5   6   7   8   9  10  11
        5'---CGATA ATG TGT TAC CTG TCT GAG GAC CAC ATG CTA G--------3'
        3'-----TAT TAC ACA ATG GAC AGA CTC CTG GTG TAC GAT CCA CG---5'
              Cla I                                     Ban I
```

The Synthetic pLac Promoter used for bTP-1/oTP-1 Expression

```
     1       10      20      30      40      50      60      70    · 80      90
    ·!        !       !       !       !       !       !       !      !        !
5'-G/AATTCGAGCGCAACGCAATTAATGTGAGTTAGCTCACTCATTAGGCACCCCAGGCTTTACACTTTATGCTTCCGGCTCGTATGTTGTGTGGA
   Eco RI
```

```
     100     110     120     130     140     150     160    ·170      180
      !       !       !       !       !       !       !       !        !
ATTGTGAGCGAATAACAATTTCAAAATTAAGGAGGTATAT/CGATAATGAAACAAAA/AGCTTCTG/GTACCC/GGGTAAGTAACTAAG/GATCC-3'
                                         Cla I          Hind III  Kpn I                    Bam HI
                                                                       Sma I
```

# FIG.9

EP 0 367 063 A1

bTP-1A

Cla I  Ban I

Bam HI

coding region

Eco RI

Ssp I

Sal I

Pvu I

Pst I

pTrp-2

Ava I

Pvu II

FIG.10

Silver Stain

Western Blot

◀ 20 kDa

◀ 20 kDa

A: pTrp2
B: pTrp2-oTP-1A
C: pTrp2-oTP-1B
D: pTrp2-bTP-1A

1: + trp (100 ug/ml)
2: + 0.1%EtOH
3: - trp
4: + 1mM IAA in 0.1%EtOH

FIG.11

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y,O | 21ST ANNUAL MEETING OF THE SOCIETY FOR THE STUDY OF REPRODUCTION, Seattle, Washington, 1st - 4th August 1988, Biological Reproduction, vol. 38, suppl. 1, 1988, page 79; J.D. GODKIN et al.: "Bovine trophoblast protein-1: Purification, antibody production, uterine cell interaction and antiviral activity" * Abstract 91 * --- | 1-14 | C 12 N 15/21<br>C 12 P 21/00<br>A 61 K 37/02<br>C 07 K 13/00 |
| X,O | ANNUAL MEETING OF THE AMERICAN SOCIETY OF ANIMAL SCIENCE, New Brunswick, New Jersey, 19th - 22nd July 1988, Journal of Animal Science, vol. 66, suppl. 1, 1988, pages 415-416; S.D. HELMER et al.: "Intrauterine infusion of purified bovine trophoblast protein-1 (bTP-1) extends corpus luteum (CL) lifespan in cyclic cattle" * Abstract 469 * --- | 15,18 | |
| Y,O | IDEM --- | 16,17 | TECHNICAL FIELDS SEARCHED (Int. Cl.5)<br><br>C 12 N<br>C 12 P |
| Y,D | NATURE, vol. 330, 26th November 1987, pages 377-379; K. IMAKAWA et al.: "Interferon-like sequence of ovine trophoblast protein secreted by embryonic trophectoderm" * Whole document * --- | 1-14,16 -17 | |
| T | WO-A-8 908 706 (INRA) --- -/- | | |

The present search report has been drawn up for all claims

| Place of search | Date of completion of the search | Examiner |
|---|---|---|
| THE HAGUE | 01-02-1990 | VAN PUTTEN A.J. |

European Patent
Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | MOLECULAR ENDOCRINOLOGY, vol. 3, no. 1, January 1989, pages 127-139, The Endocrine Society, Baltimore, US; K. IMAKAWA et al.: "Molecular cloning and characterization of complementary deoxyribonucleic acids corresponding to bovine trophoblast protein-1: A comparison with ovine trophoblast protein-1 and bovine interferon-alpfa" | 1-18 | |

TECHNICAL FIELDS
SEARCHED (Int. Cl.5)

The present search report has been drawn up for all claims

| Place of search | Date of completion of the search | Examiner |
|---|---|---|
| THE HAGUE | 01-02-1990 | VAN PUTTEN A.J. |